(19) Europäisches Patentamt
European Patent Office
Office européen des brevets

(11) **EP 4 174 081 A1**

(12) **EUROPEAN PATENT APPLICATION**
published in accordance with Art. 153(4) EPC

(43) Date of publication:
**03.05.2023 Bulletin 2023/18**

(21) Application number: **21834207.9**

(22) Date of filing: **29.06.2021**

(51) International Patent Classification (IPC):
*C07K 14/78* (2006.01)     *A61K 47/42* (2017.01)
*A61K 9/10* (2006.01)     *A61P 35/00* (2006.01)

(52) Cooperative Patent Classification (CPC):
**A61K 9/10; A61K 47/42; A61P 35/00; C07K 14/78**

(86) International application number:
**PCT/KR2021/008154**

(87) International publication number:
**WO 2022/005155 (06.01.2022 Gazette 2022/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.06.2020 KR 20200079224**

(71) Applicant: **Interoligo Corporation
Gyeonggi-do 14058 (KR)**

(72) Inventors:
• **LEE, Jung, Hwan
Yongin-si Gyeonggi-do 16803 (KR)**
• **LEE, Se, Na
Anyang-si, Gyeonggi-do 14016 (KR)**
• **KIM, Do, Young
Uiwang-si, Gyeonggi-do 16004 (KR)**

• **LEE, Da, Gyeong
Goyang-si, Gyeonggi-do 10303 (KR)**
• **LEE, Jong, Ook
Seongnam-si, Gyeonggi-do 13457 (KR)**
• **CHOI, Ji, Ah
Uiwang-si, Gyeonggi-do 16003 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

Remarks:
The complete document including Reference
Table(s) and the Sequence Listing(s) can be
downloaded from the EPO website

(54) **NOVEL ATELOCOLLAGEN AND USE THEREOF**

(57)     The present invention relates to novel atelocollagen and use thereof. More particularly, the atelocollagen is characterized in that, when analyzed by high-performance liquid chromatography (HPLC), a peak area $(S_\beta)$ for a $\beta$ chain is larger than a second peak area for an $\alpha$ chain $(S_{\alpha 2})$ so that, when a physiologically active material is loaded therein, the atelocollagen may decrease rapid initial release of the physiologically active material or may control reduction in effects of the physiologically active material due to too slow initial release. Further, the atelocollagen of the present invention may exhibit excellent cancer metastasis inhibitory effects.

EP 4 174 081 A1

[FIG. 4]

```
┌─────────────────────────────────────────────────────────────┐
│                     Fragment pig skin                        │
└─────────────────────────────────────────────────────────────┘
                              │
                              ▼
┌─────────────────────────────────────────────────────────────┐
│                      Obtain dermis                           │
└─────────────────────────────────────────────────────────────┘
                              │
                              ▼
┌─────────────────────────────────────────────────────────────┐
│          Homogenize dermis while adding acetic acid          │
└─────────────────────────────────────────────────────────────┘
                              │
                              ▼
┌─────────────────────────────────────────────────────────────┐
│          Treat homogenized dermis sample with pepsin         │
└─────────────────────────────────────────────────────────────┘
                              │
                              ▼
┌─────────────────────────────────────────────────────────────┐
│             Inactivate pepsin from dermis sample             │
└─────────────────────────────────────────────────────────────┘
                              │
                              ▼
┌─────────────────────────────────────────────────────────────┐
│         Adjust pH of sample containing inactivated pepsin    │
└─────────────────────────────────────────────────────────────┘
                              │
                              ▼
┌─────────────────────────────────────────────────────────────┐
│                     Collect supernatant                      │
│          from pH-adjusted dermis sample and salt out         │
└─────────────────────────────────────────────────────────────┘
                              │
                              ▼
┌─────────────────────────────────────────────────────────────┐
│          Wash precipitate obtained during salting out        │
└─────────────────────────────────────────────────────────────┘
                              │
                              ▼
┌─────────────────────────────────────────────────────────────┐
│       Dissolve washed precipitate in solvent and filter solution │
└─────────────────────────────────────────────────────────────┘
```

**Description**

[Technical Field]

The present invention relates to a novel atelocollagen and uses thereof.

[Background Art]

Collagen is an extracellular matrix found in the dermis, ligaments, and bones, and accounts for about 30% of the total protein in the human body. Collagen can form a basic structure of tissues or organs by gathering cells in a certain order around them. Therefore, there are many cases, in which collagen is used as a basic matrix of an artificial tissue substitute at a damaged site in order to regenerate the damaged site of the living body. In addition, the type of collagen is different depending on the tissue in which it is located. The most abundant type of collagen is Type I collagen, which has a molecular weight of about 300 kDa and consists of three polypeptides. Type 1 collagen is most widely used in tissue engineering because it is contained in large amounts in almost all tissues such as skin, ligaments, bone and the like. The molecular structure of collagen includes a non-helical telopeptide region and a triple-helical region located in N-terminal and C-terminal regions. The triple helical region is conserved across species, showing low immunogenicity, while the telopeptide region is highly immunogenic. Therefore, in order to use collagen as a raw material for pharmaceuticals or cosmetics, atelocollagen is used, which is treated with protease to remove the telopeptide region and maintains properties similar to the collagen.

In the case of using atelocollagen prepared by the conventional method as a drug carrier (generally, a drug delivery system), i) the loaded drug is unevenly loaded on the surface at a high concentration, and ii) when the drug is loaded and transplanted into the body, the drug is initially and excessively released and the drug release is rapidly reduced over time, hence causing a problem of a shorter duration of the drug. In the case of drug delivery systems, it is important to minimize side effects while maximizing therapeutic effects. For this purpose, it is important that the drug is uniformly released for a long time at an optimal drug release rate. In this regard, there is a disadvantage that the therapeutic effect of the conventional atelocollagen drug delivery system cannot be expected due to excessive initial release of the drug.

[Summary of Invention]

[Problems to be Solved by Invention]

An object of the present invention is to provide a novel atelocollagen and a method for producing the same.

In addition, another object of the present invention is to provide a drug delivery system containing the novel atelocollagen.

Further, another object of the present invention is to provide a cancer metastasis inhibitor including the novel atelocollagen.

[Means for Solving Problems]

1. An atelocollagen, including: an $\alpha$ chain containing a repeat unit of Structural Formula 1 or Structural Formula 2 below; a $\beta$ chain as a dimer of the $\alpha$ chain; and

a $\gamma$ chain as a trimer of the $\alpha$ chain, wherein, when analyzed by high-performance liquid chromatography (HPLC), a peak area ($S_\beta$) for the $\beta$ chain is larger than a second peak area for the $\alpha$ chain ($S_{\alpha2}$):

[Formula 1]        Gly-Pro-X

(in Structural Formula 1, Gly is glycine, Pro is proline, and X is an amino acid residue other than glycine and proline)

[Structural Formula 2]        Gly-Y-Hyp

(in Structural Formula 2, Gly is glycine, Y is an amino acid residue other than glycine and hydroxyproline, and Hyp is hydroxyproline).

2. The atelocollagen according to the above1, wherein the $\alpha$ chain is at least one among an $\alpha$1 chain or an $\alpha$2 chain, the $\beta$ chain is a dimer of the $\alpha$1 chain and $\alpha$1 chain, a dimer of the $\alpha$1 chain and $\alpha$2 chain, or a dimer of the $\alpha$2 chain and $\alpha$2 chain.

3. The atelocollagen according to the above1, wherein the $\alpha$ chain is at least one of $\alpha$1 chain or $\alpha$2 chain, and the $\gamma$ chain is a trimer of the $\alpha$1 chain, $\alpha$1 chain and $\alpha$2 chain.

4. The atelocollagen according to the above1, wherein the $S_\beta$ and $S_{\alpha2}$ satisfy the following Equation 1:

[Equation 1]

$$1.1 \leq S_\beta / S_{\alpha2} \leq 5$$

5. The atelocollagen according to the above1, wherein the $S_\beta$ satisfies the following Equation 2:

[Equation 2]

$$0.2 \leq S_\beta / (S_{\alpha1} + S_{\alpha2} + S_\beta + S_\gamma) \leq 0.5$$

(wherein $S_{\alpha1}$ is a first peak area for the $\alpha$ chain and $S_\gamma$ is a peak area for the $\gamma$ chain).

6. The atelocollagen according to the above1, wherein the $\alpha$ chain has a molecular weight of 100 kDa to150 kDa.

7. The atelocollagen according to the above1, wherein the high-performance liquid chromatography (HPLC) analysis is performed under conditions shown in Table 1 below:

[TABLE 1]

| Column | Reverse phase C18 column filled with silica fixed phase having octadecyl functional group bonded thereto |
|---|---|
| Mobile phase A buffer | 0.1% trifluoroacetic acid (TFA) in distilled water |
| Mobile phase B buffer | 0.1% trifluoroacetic acid in acetonitrile |
| Detection wavelength | UV 220 nm |
| Flow rate | 1 ml/min |

8. The atelocollagen according to the above7, wherein the $S_\beta$ is a value obtained by integrating peaks at an elution time of 13.0 to 13.7 minutes in a high-performance liquid chromatography (HPLC) graph.

9. The atelocollagen according to the above7, wherein the $S_{\alpha2}$ is a value obtained by integrating peaks at an elution time of 13.7 to 14.2 minutes in a high-performance liquid chromatography (HPLC) chromatogram.

10. The atelocollagen according to the above1, wherein the atelocollagen is obtained by treating a mammalian-derived dermis with a protease at 1 to 10 °C for 0.5 to 12 hours.

11. The atelocollagen according to the above10, wherein the protease is used for treatment in an amount of $0.1 \times 10^7$ unit to $3.0 \times 10^7$ unit per 100 g of dermis.

12. The atelocollagen according to the above1, wherein the atelocollagen is prepared by a method including:

treating a mammalian-derived dermis with a protease;
adding a basic solution to the dermis treated with the protease and adjusting pH in a range of 8 to 9, so as to inactivate the protease; and
pH adjustment step of treating the sample, in which the protease was inactivated, with an acidic solution to regulate the pH of the sample to 3 to 4.

13. A drug delivery system, including: the atelocollagen according to any one of the above1 to 12; and a drug loaded in the atelocollagen.

14. The drug delivery system according to the above13, wherein the atelocollagen is a structure having a plurality of pores, wherein the pore has an average diameter of 10 to 100 μm.

15. The drug delivery system according to the above13, wherein the drug delivery system is transplanted in or injected into a remaining cancer site after surgery.

16. A cancer metastasis inhibitor, including: the atelocollagen according to any one of the above1 to 12; and an anticancer agent loaded in the atelocollagen.

17. The cancer metastasis inhibitor according to the above16, wherein the cancer is pancreatic cancer, breast cancer, liver cancer, colorectal cancer, ovarian cancer, head and neck cancer or glioblastoma.

18. The cancer metastasis inhibitor according to the above 16, wherein the cancer metastasis inhibitor is transplanted in or injected into the remaining cancer site after surgery.

19. The cancer metastasis inhibitor according to the above16, wherein the anticancer agent is a low molecular weight compound, high molecular weight compound, nucleic acid, peptide, protein or aptamer.

[Advantageous effects]

Atelocollagen of the present invention may exhibit different physical properties (e.g., excellent viscosity) from conventional atelocollagen since it has a higher content of β chains than α 2 chains.

A physiologically active material may be uniformly loaded in the atelocollagen of the present invention.

When the physiologically active material is loaded in the atelocollagen of the present invention, a decrease in effects of the physiologically active material due to the too slow initial release of the physiologically active material in the body may be controlled. That is, when the physiologically active material is loaded in the atelocollagen of the present invention, the release rate of the physiologically active material in the body may be controlled, and ultimately, the efficacy of the physiologically active material in the body may be increased by controlling the release of the physiologically active material.

The atelocollagen of the present invention may exhibit an excellent cancer metastasis inhibitory effect.

The atelocollagen of the present invention may be biodegradable.

The production method of the present invention may adjust the content of α chain, β chain or γ chain of atelocollagen.

[Brief Description of Drawings]

FIG. 1 shows an example of a high-performance liquid chromatography (HPLC) analysis chart (chromatogram).

FIG. 2 shows an example of a high-performance liquid chromatography (HPLC) analysis chart (chromatogram).

FIG. 3 shows an example of a high-performance liquid chromatography (HPLC) analysis graph (chromatogram) according to the analysis conditions of an embodiment.

FIG. 4 shows a flowchart of a method for production of atelocollagen according to an embodiment.

FIG. 5 illustrates a method of substituting atelocollagen-sodium acetate solution with atelocollagen-buffer using a tangential flow filtration system (Minimate TFF system).

FIG. 6 illustrates a mechanism of cancer metastasis inhibitory effect of atelocollagen according to an embodiment.

FIG. 7 illustrates a mechanism of cancer metastasis inhibitory effect of atelocollagen according to Comparative Example.

FIG. 8 shows results of confirming the purity of a pH neutral atelocollagen solution through SDS-PAGE.

FIG. 9 shows results of confirming the distribution of atelocollagen chains in the HPLC analysis chromatogram by prepping peaks of atelocollagen by HPLC elution time, and then analyzing the same through SDS-PAGE.

FIG. 10 shows graphs obtained by HPLC analysis of the atelocollagen in examples and the atelocollagen (commercially available) in comparative examples, respectively, under the same conditions.

FIG. 11 shows graphs indicating numerical values of an area of each peak in the graphs obtained by HPLC analysis of the atelocollagens in the examples and comparative examples under the same conditions.

FIG. 12 shows graphs obtained by HPLC analysis of the atelocollagens in the examples and comparatives under the same conditions.

FIG. 13 shows graphs indicating numerical values of the area of each peak in the graphs obtained by HPLC analysis of the atelocollagens in the examples and comparative examples under the same conditions.

FIG. 14 shows graphs indicating numerical values of the area of each peak in the graphs obtained by HPLC analysis of the atelocollagens in the examples and comparative examples under the same conditions.

FIG. 15 shows graphs indicating numerical values of the area of each peak in the graphs obtained by HPLC analysis of the atelocollagens in the examples and comparative examples under the same conditions.

FIG. 16 shows graphs indicating numerical values of the area of each peak in the graphs obtained by HPLC analysis of the atelocollagens in the examples and comparative examples under the same conditions.

FIG. 17 shows graphs indicating numerical values of the area of each peak in the graphs obtained by HPLC analysis of the atelocollagens in the comparative examples under the same conditions.

FIG. 18 shows graphs indicating the numerical values of the area of each peak in the graph obtained by HPLC analysis of the atelocollagens in the examples under the same conditions.

FIG. 19 shows graphs indicating numerical values of the area of each peak in the graphs obtained by HPLC analysis of the atelocollagens in the examples under the same conditions.

FIG. 20 shows graphs indicating numerical values of the area of each peak in the graphs obtained by HPLC analysis of the atelocollagens in the examples under the same conditions.

FIG. 21 shows graphs indicating numerical values of the area of each peak in the graphs obtained by HPLC analysis of the atelocollagens in the examples under the same conditions.

FIG. 22 shows graphs indicating the numerical value of the area of each peak in the graphs obtained by HPLC analysis of the atelocollagens in the examples under the same conditions.

FIG. 23 shows graphs indicating the numerical value of the area of each peak in the graphs obtained by HPLC analysis of the atelocollagens in the examples under the same conditions.

FIG. 24 shows graphs indicating numerical values of the area of each peak in the graphs obtained by HPLC analysis of the atelocollagens in the examples under the same conditions.

FIG. 25 shows results of analysis of the atelocollagens in the examples and comparative examples by sodium dodecyl sulfate polyacrylamide gel electrophoresis (SDS-PAGE).

FIG. 26 shows results of observing structures of the atelocollagens in the examples and comparative examples using a scanning electron microscope.

FIG. 27 shows results of observing the structures of the atelocollagens in the examples and comparative examples, in each of which the drug was loaded, using a scanning electron microscope.

FIG. 28 is a graph and detailed numerical values for tensile strength test results of the atelocollagen in Example 1.

FIG. 29 is a graph and detailed numerical values of tensile strength test results of the atelocollagen in Example 31.

FIG. 30 is a graph and detailed numerical values of tensile strength test results of the atelocollagen in Comparative Example 2.

FIG. 31 illustrates steps of preparing a solid atelocollagen drug delivery system (including patch type atelocollagen drug delivery system).

FIG. 32 shows results of confirming MMP-2 and MMP-9 activity by means of Zymography.

FIG. 33 shows graphs of comparing drug release effects between the atelocollagen drug delivery system of the comparative example and the atelocollagen drug delivery system of the examples in a medium in which MMP-2 and MMP-9 are overexpressed.

FIG. 34 shows tumor suppression and metastasis inhibitory effects of a patch type atelocollagen drug delivery system loaded with gemcitabine.

FIG. 35 shows tumor suppression and metastasis inhibitory effects of a patch type atelocollagen drug delivery system loaded with [GRO-aptamer and gemcitabine conjugate].

FIG. 36 shows tumor suppression and metastasis inhibitory effects of a sol-gel type atelocollagen drug delivery system loaded with gemcitabine.

FIG. 37 shows tumor suppression and metastasis inhibitory effects of a sol-gel type atelocollagen drug delivery system loaded with [GRO-aptamer and gemcitabine conjugate].

FIG. 38 shows graphs confirming drug release effects in the patch type atelocollagen drug delivery system loaded with [GRO-aptamer and gemcitabine conjugate].

[Mode for Carrying out Invention]

Hereinafter, the present invention will be described in detail.

The present invention provides a novel atelocollagen.

The present invention provides atelocollagen which includes an $\alpha$ chain; a $\beta$ chain as a dimer of the $\alpha$ chain; and a $\gamma$ chain as a trimer of the $\alpha$ chain, wherein, when analyzed by high-performance liquid chromatography (HPLC), a peak area ($S_\beta$) for the $\beta$ chain is larger than a second peak area for the $\alpha$ chain ($S_{\alpha 2}$).

The term "area of a peak" means the area of a ridge having a corresponding peak present in an HPLC analysis graph, wherein the ridge having each peak and its area are defined according to a conventional chromatographic analysis method.

The area of the peak may mean a value obtained by integrating a detected amount from an elution time at an inflection point immediately before the elution time corresponding to a specific peak (or an elution time at an intersection of the ridge and a baseline immediately before the elution time corresponding to the specific peak) to an elution time at the inflection point immediately after the elution time corresponding to the above peak (or an elution time at the intersection of the ridge and the baseline immediately after the elution time corresponding to the peak), in the HPLC analysis chart (chromatogram). Herein, the baseline refers to a line parallel to a width (horizontal) axis with the detection value of 0 in the HPLC analysis chart.

A method of calculating the area of a ridge having a specific peak will be described with the following example. However, the area of the peak having a specific peak is not particularly limited to the following example, but may also be obtained according to any conventional chromatographic analysis method.

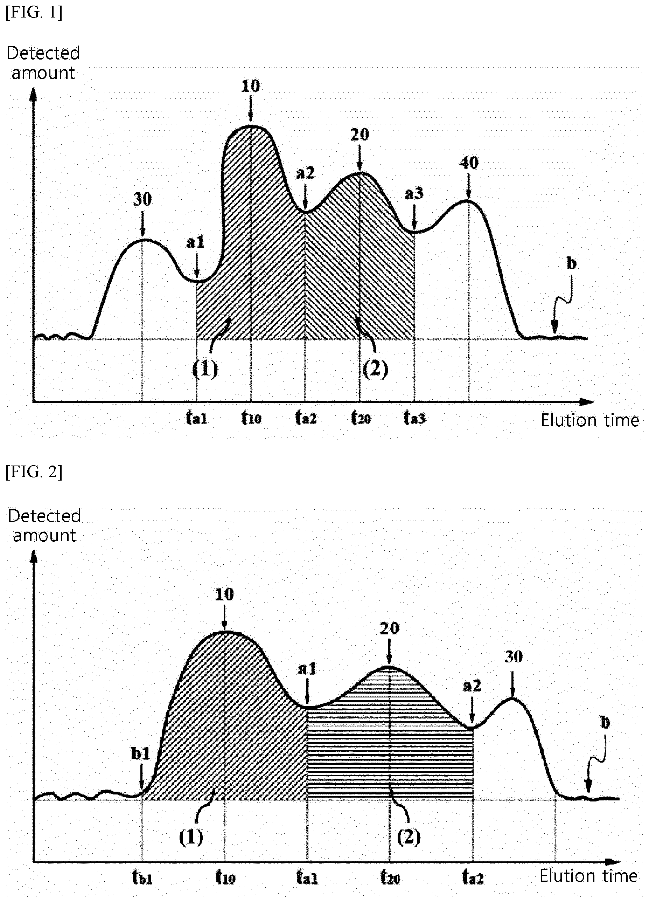

In the case where: a ridge having a different peak before the elution time of the specific peak, for which the area is to be calculated (at this time, the ridge having the above other peak is adjacent to the ridge having the specific peak, and there is an inflection point between the ridge having the other peak and the ridge having the specific peak) is present; and another ridge having another peak after the elution time of the specific peak (at this time, the ridge having another peak is adjacent to the ridge having the specific peak, and there is an inflection point between the ridge having another peak and the ridge having the specific peak) is present, the method of calculating the area of the ridge having the specific peak will be described in detail with reference to FIG. 1.

In FIG. 1, the area of a ridge having a first peak 10 means a value obtained by integrating a detected amount from an elution time (ta1) corresponding to a first inflection point (a1) present between the ridge having a third peak 30, the elution time of which is faster than the first peak 10, and the ridge having the first peak 10 to an elution time (ta2) corresponding to a second inflection point (a2) present between the ridge having a second peak 20, the elution time of which is slower than the first peak 10, and the ridge having the first peak 10 (see FIG. 1) (at this time, the third peak 30 is present before the first peak 10, and the ridge having the third peak 30 is adjacent to the ridge having the first peak 10. Further, the second peak 20 is present after the first peak, and the ridge having the second peak 20 is adjacent to the ridge having the first peak 10). That is, this demonstrates an area 1 of an oblique portion inclined downward to the left in FIG. 1.

Similarly, referring to FIG. 1, the area of the ridge having the second peak 20 means a value obtained by integrating a detected amount from the elution time (ta2) corresponding to the second inflection point (a2) present between the ridge having the first peak 10, the elution time of which is faster than the second peak 20, and the ridge having the second peak 20 to an elution time (ta3) corresponding to a third inflection point (a3) present between the ridge having a fourth peak 40, the elution time of which is slower than the second peak 20, and the ridge having the second peak 20 (see FIG. 1) (at this time, the first peak 10 is present before the second peak 20, and the ridge having the first peak 10 is adjacent to the ridge having the second peak 20. Further, the fourth peak 40 is present after the second peak 20, and the ridge having the fourth peak 40 is adjacent to the ridge having the second peak 20). That is, this area means an area 2 of the oblique portion inclined downward to the right in FIG. 1.

If there is an intersection of the ridge having a specific peak and a baseline before an elution time of the specific peak, for which the area is to be calculated (at this time, no other peak exists between the intersection and the specific peak), and when there is a ridge having another peak after the elution time of the specific peak (at this time, the ridge having the another peak is adjacent to the ridge having the specific peak, and an inflection point exists between the ridge having the another peak and the ridge having the specific peak), a method of calculating an area of the ridge having the specific peak (corresponding to the first peak 10 in FIG. 2) will be described in detail with reference to FIG. 2.

In FIG. 2, the area of the ridge having the first peak 10 means a value obtained by integrating a detected amount from an elution time (tb 1) corresponding to an intersection (b 1) of a baseline b appearing at a time earlier than an elution time (t10) of the first peak 10 and the ridge having the first peak 10, to the elution time (ta1) corresponding to the first inflection point (a1) present between the ridge having the second peak 20, the elution time of which is slower than the first peak 10, and the ridge having the first peak 10 (see FIG. 2) (at this time, no other peak exists between the intersection (b 1) and the first peak 10, the second peak 20 is present after the first peak 10, and the ridge having the second peak 20 is adjacent to the ridge having the first peak 10). That is, the above area means an area 1 of the oblique portion inclined downward to the left in FIG. 2.

Meanwhile, referring to FIG. 2, the area of the ridge having the second peak 20 means a value obtained by integrating a detected amount from the elution time (ta1) corresponding to the first inflection point (a1) present between the ridge having the first peak 10 and the ridge having the second peak 20 to the elution time (ta2) corresponding to the second inflection point (a2) present between the area having a third peak 30, the elution time of which is slower than the second peak 20, and the ridge having the second peak 20 (see FIG. 2) (at this time, the first peak 10 is present before the second peak 20 and the ridge having the first peak 10 is adjacent to the ridge having the second peak 20. Further, the third peak 30 is present after the second peak 20, and the ridge having the third peak 30 is adjacent to the ridge having the second peak 20). That is, the above area indicates an area 2 of a portion indicated by lines parallel to the horizontal axis in FIG. 2.

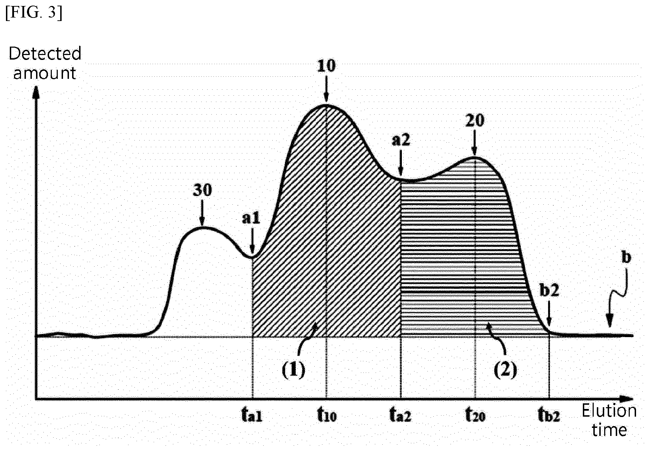

After the elution time of the specific peak for which the area is to be calculated, when there is an intersection of the ridge having the specific peak and the baseline (at this time, no other peak exists between the intersection and the specific peak), and when a ridge having another peak before the elution time of the specific peak is present (at this time, the ridge having another peak is adjacent to the ridge having the specific peak, and there is an inflection point present between the ridge having the another peak and the ridge having the specific peak), a method of calculating an area of the ridge having the specific peak (corresponding to the second peak 20 in FIG. 3) will be described in detail with reference to FIG. 3.

In FIG. 3, the area of the ridge having the second peak 20 means a value obtained by integrating a detected amount from the elution time (ta2) corresponding to the second inflection point (a2) present between the ridge having the first peak 10, the elution time of which is faster than the second peak 20, and the ridge having the second peak 20, to an elution time (tb2) corresponding to an intersection (b2) of the baseline b and the ridge having the second peak 20 at a time slower than an elution time (t20) of the second peak 20 (see FIG. 3) (at this time, no other peak exists between the intersection (b2) and the second peak 20. Further, the first peak 10 is present before the second peak 20, and the ridge having the first peak 10 is adjacent to the ridge having the second peak 20). That is, the above area refers to an area 2 of a portion indicated by lines parallel to the horizontal axis in FIG. 3.

Meanwhile, referring to FIG. 3, the area of the ridge having the first peak 10 means a value obtained by integrating a detected amount from the elution time (ta1) corresponding to the first inflection point (a1) present between the ridge having the first peak 10, the elution time of which is faster than the second peak 20, and the ridge having the second peak 20, to the elution time (ta2) corresponding to the second inflection point (a2) present between the ridge having the second peak 20, the elution time of which is slower than the first peak 10, and the ridge having the first peak 10 (see FIG. 3) (at this time, the third peak 30 is present before the first peak 10, and the ridge having the third peak 30 is adjacent to the ridge having the first peak 10. Further, the second peak 20 is present after the first peak 10, and the ridge having the second peak 20 is adjacent to the ridge having the first peak 10). That is, the above area means an area 1 of the oblique portion inclined downward to the left in FIG. 3.

The area of each peak is proportional to a content of the material representing the peak. That is, with regard to the atelocollagen of the present invention, the content of the β chain included in the atelocollagen is higher than the content of the α2 chain. Specifically, the unit of the content may be w/v %.

The term "atelocollagen" refers to the removal of telopeptide located at the N-terminus and C-terminus of collagen, wherein the immunogenicity is removed while maintaining the characteristics of collagen. Atelocollagen may be prepared by removing N-terminal and C-terminal telopeptides from naturally existing collagen. For example, the atelocollagen of the present invention may be prepared by removing the N-terminal and C-terminal telopeptides from Type 1 or Type 2 collagen.

The term "collagen" refers to a protein that occupies most of the extracellular matrix, and is involved in the formation of tissue structures or interactions between cells or between cell and matrix. A collagen molecule is a molecule in which three strands of the α chain as a basic structural unit are twisted to form a helix structure, wherein the α chain includes Gly-AB repeat unit (Gly is glycine, A and B are amino acids, and at least one of A and B is proline or hydroxyproline), and may be a peptide consisting of about 1000 amino acids. There are many different types of collagen depending on function, arrangement, etc. in the tissue. Representative types of collagen may include Type 1 collagen and Type 2 collagen. Type 1 collagen consists of two α1 chains and one α2 chain, while Type 2 collagen consists of three α1 chains.

The α chain included in the atelocollagen of the present invention may include Gly-A-B repeat unit (Gly is glycine, A and B are amino acids, and at least one of A and B is proline or hydroxyproline).

For example, the α chain included in atelocollagen of the present invention may include a repeat unit of the following Structural Formula 1 or Structural Formula 2:

[Structural Formula 1]        Gly-Pro-X

(in Structural Formula 1, Gly is glycine, Pro is proline, and X is an amino acid residue other than glycine and proline),

[Structural Formula 2]        Gly-Y-Hyp

(in Structural Formula 2, Gly is glycine, Y is an amino acid residue other than glycine and hydroxyproline, and Hyp is hydroxyproline).

The α chain may be at least one of an α1 chain or an α2 chain.

The β chain may be a dimer of the α1 chain and the α1 chain, a dimer of the α1 chain and the α2 chain, or a dimer of the α2 chain and the α2 chain.

The γ chain may be a trimer of the α1 chain, the α1 chain and the α2 chain.

The α chain may have a molecular weight of 100 kDa to 150 kDa, such as 100 kDa, 105 kDa, 110 kDa, 115 kDa, 120 kDa, 125 kDa, 130 kDa, 131 kDa, 132 kDa, 133 kDa, 134 kDa, 135 kDa, 136 kDa, 137 kDa, 138 kDa, 139 kDa, 140 kDa, 145 kDa or 150 kDa.

The β chain may have a molecular weight of 180 kDa to 270 kDa.

The γ chain may have a molecular weight of 280 kDa to 350 kDa.

The atelocollagen of the present invention may further include ancillary components (e.g., water, salt, buffer, etc.) in addition to the $\alpha$ chain, $\beta$ chain and $\gamma$ chain.

When the atelocollagen of the present invention is analyzed by high-performance liquid chromatography (HPLC), $S_\beta$ and $S_{\alpha2}$ may satisfy the following Equation 1:

[Equation 1]

$$1.1 \leq S_\beta / S_{\alpha2} \leq 5.$$

When analyzing the atelocollagen of the present invention by high-performance liquid chromatography (HPLC), the $S_\beta / S_{\alpha2}$ value may be 1 to 10, 1 to 9, 1 to 8, 1 to 7, 1 to 6, 1 to 5, 1.1 to 5, 1.1 to 4.5, or 1.2 to 4.5. When analyzing the atelocollagen of the present invention by high-performance liquid chromatography (HPLC), the $S_\beta / S_{\alpha2}$ value may be 1.1 to 5.0, 1.5 to 4.5, 2.0 to 4.0, or 2.5 to 3.5. When analyzing the atelocollagen of the present invention by high-performance liquid chromatography (HPLC), the $S_\beta / S_{\alpha2}$ value may be 1.1 to 1.9, 2.0 to 2.9, or 3.0 to 4.5.

When analyzing the atelocollagen of the present invention by high-performance liquid chromatography (HPLC), $S_\beta / S_{\alpha2}$ may satisfy the following equations: $1 \leq S_\beta / S_{\alpha2} \leq 5$, $1.1 \leq S_\beta / S_{\alpha2} \leq 4.5$, $1.2 \leq S_\beta / S_{\alpha2} \leq 4.5$, $1.5 \leq S_\beta / S_{\alpha2} \leq 4.5$, $2.0 \leq S_\beta / S_{\alpha2} \leq 4.0$, $2.5 \leq S_\beta / S_{\alpha2} \leq 3.5$, or $2.0 \leq S_\beta / S_{\alpha2} \leq 3.0$.

When analyzing the atelocollagen of the present invention by high-performance liquid chromatography (HPLC), $S_\beta$ may satisfy the following Equation 2:

[Equation 2]

$$0.2 \leq S_\beta / (S_{\alpha1} + S_{\alpha2} + S_\beta + S_\gamma) \leq 0.5$$

wherein $S_{\alpha1}$ is a first peak area for the $\alpha$ chain and $S_\gamma$ is a peak area for the $\gamma$ chain). The $(S_{\alpha1} + S_{\alpha2} + S_\beta + S_\gamma)$ refers to a total area of respective peaks derived from atelocollagen when analyzing the atelocollagen by high-performance liquid chromatography (HPLC).

When analyzing the atelocollagen of the present invention by high-performance liquid chromatography (HPLC), $S_\beta / (S_{\alpha1} + S_{\alpha2} + S_\beta + S_\gamma)$ may satisfy the following equations: $0.2 \leq S_\beta / (S_{\alpha1} + S_{\alpha2} + S_\beta + S_\gamma) \leq 0.5$, $0.2 \leq S_\beta / (S_{\alpha1} + S_{\alpha2} + S_\beta + S_\gamma) \leq 0.4$, $0.2 \leq S_\beta / (S_{\alpha1} + S_{\alpha2} + S_\beta + S_\gamma) \leq 0.3$, or $0.2 \leq S_\beta / (S_{\alpha1} + S_{\alpha2} + S_\beta + S_\gamma) \leq 0.25$.

When analyzing the atelocollagen of the present invention by high-performance liquid chromatography (HPLC), $S_\beta / (S_{\alpha1} + S_{\alpha2} + S_\beta + S_\gamma)$ may satisfy the following equations: $0.2 \leq S_\beta / (S_{\alpha1} + S_{\alpha2} + S_\beta + S_\gamma) \leq 0.3$, $0.3 < S_\beta / (S_{\alpha1} + S_{\alpha2} + S_\beta + S_\gamma) \leq 0.4$, $0.4 < S_\beta / (S_{\alpha1} + S_{\alpha2} + S_\beta + S_\gamma) \leq 0.5$.

The first peak for the $\alpha$ chain is a peak for the $\alpha1$ chain, while the second peak for the $\alpha$ chain is a peak for the $\alpha2$ chain. The order of the peaks for the $\alpha$ chain is distinguished based on the elution time. That is, the elution time of the first peak for the $\alpha$ chain is faster than the elution time of the second peak for the $\alpha$ chain.

When analyzing the atelocollagen of the present invention by high-performance liquid chromatography (HPLC), $S_\beta / (S_{\alpha1} + S_{\alpha2} + S_\beta + S_\gamma)$ may be 0.1 to 0.5, 0.11 to 0.45, 0.12 to 0.45, or 0.13 to 0.45. For example, when analyzing the atelocollagen of the present invention by high-performance liquid chromatography (HPLC), $S_\beta / (S_{\alpha1} + S_{\alpha2} + S_\beta + S_\gamma)$ may be 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, or 0.5.

When analyzing the atelocollagen of the present invention by high-performance liquid chromatography (HPLC), $S_{\alpha2} / (S_{\alpha1} + S_{\alpha2} + S_\beta + S_\gamma)$ may be 0.05 to 0.3 or 0.05 to 0.25. For example, when analyzing the atelocollagen of the present invention by high-performance liquid chromatography (HPLC), Saz / $(S_{\alpha1} + Saz + S_\beta + S_\gamma)$ may be 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, or 0.3.

For high-performance liquid chromatography (HPLC) analysis, a reversed-phase C18 column filled with a silica fixed phase to which an octadecyl functional group is bound may be used, but the type thereof is not limited as long as it can analyze the sample.

According to an embodiment, high-performance liquid chromatography (HPLC) analysis may be performed under the conditions of Table 1 below.

[TABLE 1]

| Column | Reverse phase C18 column filled with silica fixed phase having octadecyl functional group bonded thereto |
|---|---|
| Mobile phase A buffer | 0.1% trifluoroacetic acid (TFA) in distilled water |
| Mobile phase B buffer | 0.1% trifluoroacetic acid in acetonitrile |
| Detection wavelength | UV 220 nm |
| Flow rate | 1 ml/min |

The high-performance liquid chromatography analysis conditions may be varied for optimal analysis, but these do not change the properties of the atelocollagen of the present invention. The graph obtained as a result of high-performance liquid chromatography analysis may demonstrate the detected amount or the detection intensity on the vertical axis, and the elution time on the horizontal axis.

According to an embodiment, $S_\beta$ may be the area of a ridge having a peak with an elution time of 13.0 to 13.7 minutes in the high-performance liquid chromatography graph performed under the conditions of Table 1. According to an embodiment, $S_{\alpha 2}$ may be the area of a ridge having a peak with an elution time of 13.7 to 14.2 minutes in the high-performance liquid chromatography graph performed under the conditions of Table 1.

As described above, the $\beta$ chain content of the atelocollagen of the present invention is higher than the $\alpha 2$ chain content. However, in the conventional atelocollagen, the $\beta$ chain content compared to the total atelocollagen is lower than the $\alpha 2$ chain content (in the case of conventional atelocollagen, a peak area of the $\beta$ chain ($S_\beta$) is equal to the area of the peak for the $\alpha 2$ chain ($S_{\alpha 2}$) or smaller than the area of the peak ($S_{\alpha 2}$) for the $\alpha 2$ chain). This may depend on to the method for production of atelocollagen according to the present invention, which will be described below, for example, depending on differences between the performing steps and the performing conditions.

The atelocollagen of the present invention may be one obtained by treating mammalian-derived dermis with a protease at 1 to 10 °C for 0.5 to 12 hours.

The atelocollagen of the present invention may be one obtained by treating mammalian-derived dermis with the protease at 18 to 30 °C for less than 3 hours.

The atelocollagen of the present invention may be obtained by treating mammalian-derived dermis with the protease and reacting the same at 1 to 15 °C for 12 hours or less, and at 18 to 30 °C for less than 3 hours.

The protease may be used for treatment in an amount of $0.1 \times 10^7$ to $3.0 \times 10^7$ unit, $0.5 \times 10^7$ to $2.5 \times 10^7$ unit, $0.8 \times 10^7$ to $2.3 \times 10^7$ unit, $1.0 \times 10^7$ to $2 \times 10^7$ unit, $1.3 \times 10^7$ to $1.8 \times 10^7$ unit per 100 g of dermis.

The atelocollagen of the present invention may be produced by a method including: treating a mammalian-derived dermis with a protease; adding a sodium hydroxide solution to the dermis treated with the protease and adjusting pH in a range of 8 to 9, so as to inactivate the protease; and pH adjustment step of treating the sample, in which the protease was inactivated, with an acidic solution to regulate the pH of the sample to 3 to 4.

The atelocollagen of the present invention may be derived from a mammal, and the mammal may be a human, pig, cow, chicken or the like. The atelocollagen of the present invention may be derived from tissues of the mammal, and the tissue may be skin, tail, cartilage, bone, tendon, ligament or the like.

The atelocollagen of the present invention may be formulated in various forms, for example, in the form of an aqueous solution, dispersion, gel, powder, tablet, patch, film, sponge, foam or matrix.

Further, the present invention provides a novel method for producing atelocollagen.

The above production method may include (a) treating a mammalian dermal sample with a protease.

In treatment step with protease, the mammal may be a human, a pig, a cow, a chicken or the like. In treatment step with protease, the dermis may be obtained from tail, cartilage, bone, tendon or ligament. The protease may be at least one of pepsin, papain, alkalase and catalase. According to an embodiment, the protease may be pepsin. The dermal sample may be homogenized while adding acetic acid to the dermis of the mammal.

In treatment step with protease, the protease may be used for treatment in an amount of: $0.1 \times 10^7$ to $3.0 \times 10^7$ unit, $0.5 \times 10^7$ to $2.5 \times 10^7$ unit, $0.8 \times 10^7$ to $2.3 \times 10^7$ unit, $1.0 \times 10^7$ to $2.0 \times 10^7$ unit, $1.3 \times 10^7$ to $1.8 \times 10^7$ unit per 100 g of the dermis. According to an embodiment, the protease may be used for treatment in an amount of $1.5 \times 10^7$ unit per 100 g of the dermis.

In treatment step with protease, the protease may be used for treatment at 1 to 30 °C, 1 to 25 °C, 1 to 15 °C, 1 to 10 °C, 1 to 8 °C, 2 to 6 °C, or 3 to 5°C. Further, according to an embodiment, the protease may be used for treatment at 4 °C. In treatment step with protease, the protease may be used for treatment for 24 hours or less. For example, the protease is used for treatment for 0.1 to 24 hours, 0.2 to 20 hours, 0.3 to 18 hours, 0.4 to 16 hours, 0.5 to 12 hours, 1 hour to 5 hours, or 1.5 to 3 hours.

In treatment step with protease, the protease may be used for treatment at 1 to 15 °C for 12 hours or less.

According to an embodiment, the protease may be used for treatment at 4 °C for 1.5 to 11.5 hours (e.g., at 4 °C for 1.5 to 3 hours, 4.5 hours, 11.5 hours). According to an embodiment, when the protease is used at 4 °C for less than 13 hours, for example, 0.5 to 12 hours, the atelocollagen of the present invention with the content of the β chain higher than the content of the α2 chain may be obtained even if the protease inactivation process is not conducted.

In treatment step with protease, the protease may be used for treatment at 18 to 30 °C for less than 3 hours. According to an embodiment, the protease may be used at 18 to 24 °C for 1.5 hours. According to an embodiment, when the protease is used for treatment at RT (room temperature) for less than 3 hours, the atelocollagen of the present invention with the content of the β chain higher than the content of the α2 chain may be obtained even if the protease inactivation process is not conducted.

In treatment step with protease, the protease may be used for treatment at 1 to 15 °C for 12 hours or less, or at 18 to 30 °C for less than 3 hours. According to an embodiment, the protease may be treated at 4 °C for 1.5 to 11.5 hours, or at RT for less than 3 hours.

As such, with regard to the atelocollagen of the present invention, the content ratio of α chain, β chain and γ chain may be adjusted according to the conditions under which the protease is used for treatment in treatment step with protease. According to an embodiment, the content ratio of the α chain, the β chain and the γ chain may be adjusted by varying the amount of pepsin used, the reaction temperature or time in treatment step with protease.

The above production method may further include: (b) inactivating the protease in the dermal sample.

The protease inactivation step may include adding a basic solution (e.g., sodium hydroxide solution) to the protease-treated dermal sample to inactivate the protease. According to an embodiment, when the protease inactivation step is conducted, it could be confirmed that the atelocollagen of the present invention having the β chain content higher than the α2 chain content can be obtained.

The protease inactivation step may be conducted at 1 to 10 °C, 1 to 8 °C, 1 to 5 °C, or 1 to 4 °C.

The protease inactivation step may be for adjusting the pH to 8 to 12, 8 to 11, 8 to 10, or 8 to 10 by adding a basic solution to the dermal sample treated with the protease.

The sodium hydroxide solution may have a concentration of 1 to 28 M, 1 to 25 M, 1 to 20 M, 1 to 15 M or 1 to 10 M.

The above production method may further include: (c) a pH adjustment step of adjusting the pH of the sample to 3 to 4 by treating the sample, in which the protease is inactivated, with an acidic solution.

As such, the atelocollagen of the present invention may be produced by the method including both the protease inactivation step and the pH adjustment step so that the content ratio of the α chain, the β chain and the γ chain may be adjusted.

The above production method may further include: (d) adding a sodium chloride solution to the supernatant of the pH-adjusted dermal sample and obtaining a precipitate.

The supernatant of the dermal sample may be one from which fat or impurities have been removed.

In the precipitate obtaining step, the supernatant may be collected by centrifuging the sample whose pH is adjusted to 3 to 4, while fat and impurities may be removed by centrifugation.

The above production method may further include: (e) washing and/or filtering the precipitate. Through the filtration step, purified atelocollagen at higher purity may be obtained.

The atelocollagen and/or purified atelocollagen obtained by the above production method may be lyophilized and stored.

According to an embodiment, the atelocollagen of the present invention may be an atelocollagen dispersion obtained by dissolving atelocollagen or purified atelocollagen prepared by the above-described method in acid, followed by diafiltration while adding a buffer.

According to an embodiment, the atelocollagen of the present invention may be an atelocollagen dispersion obtained by dissolving atelocollagen prepared by the above-described method and then lyophilized and stored in acid, followed by diafiltration while adding a buffer.

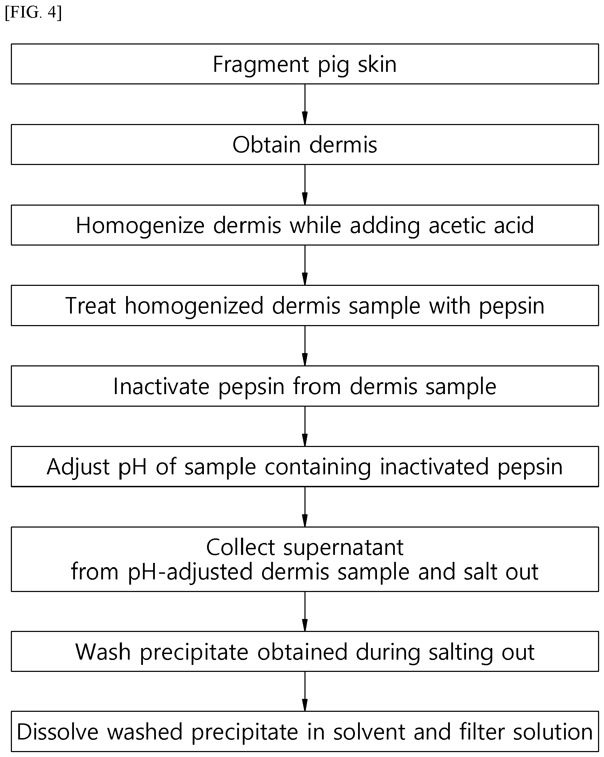

According to an embodiment, the atelocollagen of the present invention may be produced by a method including: fragmenting a pig skin; obtaining dermis from the pig skin; adding acetic acid to the obtained dermis and homogenizing the same; treating the homogenized dermal sample with pepsin; inactivating pepsin in the dermal sample; adjusting the pH of the pepsin-inactivated sample; collecting a supernatant in which fat or impurities have been removed from the pH-adjusted dermal sample; adding the sodium chloride solution to the collected supernatant and then salting out so as to obtain a precipitate; washing the obtained precipitate with ethanol; and dissolving the washed precipitate in a solvent and filtering the same (see FIG. 4).

Further, the present invention provides a drug delivery system including atelocollagen.

The drug delivery system of the present invention may include the above-described atelocollagen and a physiologically active material loaded in the atelocollagen. The drug delivery system of the present invention may be used to deliver the physiologically active material.

The drug delivery system of the present invention may be formulated in various forms, such as dispersions, gels, powders, tablets, patches, films, sponges, foams, matrices and the like.

In the drug delivery system of the present invention, atelocollagen may be a solid atelocollagen structure.

In the drug delivery system of the present invention, atelocollagen may be an atelocollagen structure having a plurality of pores.

With regard to the drug delivery system of the present invention, physical properties thereof (e.g., size of pores, degree of opening between pores, biodegradation rate, drug loading amount, drug loading pattern, drug release rate, initial drug release rate, drug release profile, etc.) may be regulated according to the content ratio of $\alpha 1$ chain, $\alpha 2$ chain, $\beta$ chain and $\gamma$ chain of the atelocollagen included in the drug delivery system.

The physical properties may be regulated to exhibit optimal drug efficacy in consideration of clinical application conditions (e.g., loaded drugs, implanted body sites, adaptation, etc.) of the drug delivery system according to the present invention.

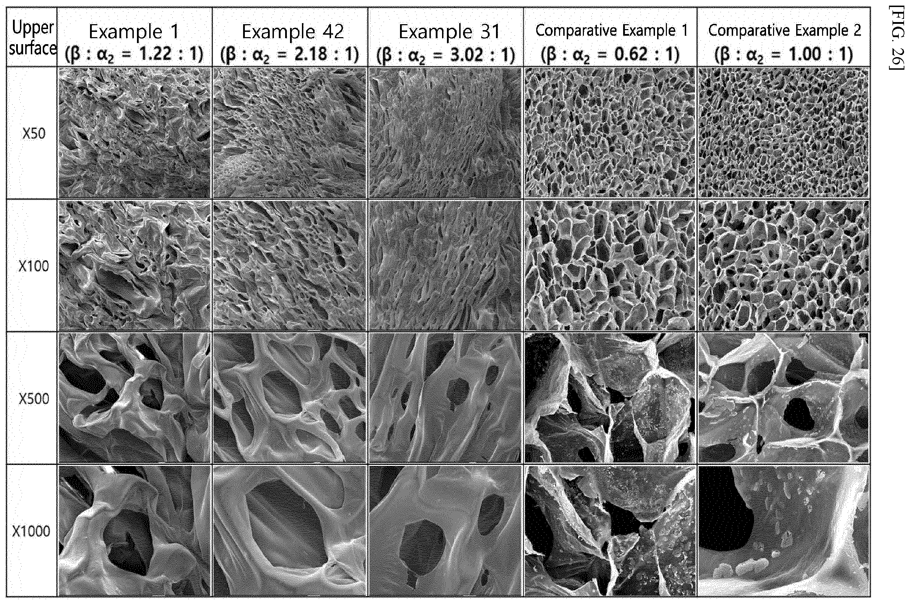

With regard to the atelocollagen structure having a plurality of pores prepared using the atelocollagen of the present invention, an average diameter of the pores is smaller than that of a structure produced using conventional atelocollagen, in which the content of $\beta$ chains is substantially equal to or smaller than the content of $\alpha 2$ chains, by the same method as described above (see Table 3 and FIG. 26). According to an embodiment, the pores may have an average diameter of 10 to 100 $\mu$m, 20 to 90 $\mu$m, 30 to 80 $\mu$m, or 3 to 75 $\mu$m. The atelocollagen structure having a plurality of pores prepared using the atelocollagen of the present invention may be a structure having open pores on both sides.

The solid atelocollagen structure prepared using atelocollagen of the present invention has a tensile strength smaller than that of a structure formed in the same way using conventional atelocollagen, in which the content of $\beta$ chains is substantially equal to or smaller than the content of $\alpha 2$ chains. According to an embodiment, an atelocollagen structure having a width of 20 mm, a length of 3 mm and a thickness of 2 mm prepared by lyophilizing the atelocollagen of the present invention is subjected to application of uniform tension at a speed of 10.0 mm/min in a longitudinal axis direction of the structure, followed by measuring the maximum tension until the material is cut out so that the measured tensile strength may range from 0.05 to 0.30 N/mm$^2$. For example, the atelocollagen structure may have a tensile strength of 0.05 to 0.30 N/mm$^2$, 0.1 to 0.25 N/mm$^2$, or 0.15 to 0.20 N/mm$^2$, which is measured according to a method of applying tension uniformly to an atelocollagen structure having a width of 20 mm, a length of 3 mm and a thickness of 2 mm prepared by lyophilizing the atelocollagen of the present invention, and then, measuring the maximum tension until the material is cut out.

The atelocollagen of the present invention has a viscous higher than the conventional atelocollagen, in which the area ($S_{\beta}$) of a peak for the $\beta$ chain is equal to or smaller than the area ($S_{\alpha 2}$) of a peak for the $\alpha 2$ chain. According to an embodiment, the atelocollagen may have a viscosity of 1.0 to 3.0 Pa s, which is measured according to an analysis method using a rotational rheometer on an atelocollagen solution prepared to have a collagen concentration of 1.0% and pH 6.8 to 7.4 (neutral) using the atelocollagen of the present invention. For example, the viscosity measured according to the analysis method using the rotational rheometer on an atelocollagen solution prepared to have a collagen concentration of 1.0% and a pH of 6.8 to 7.4 (neutral) using the atelocollagen of the present invention may be 1.0 to 3.0 Pa s, 1.5 to 2.5 Pa s, or 1.8 to 2.0 Pa s.

The solid atelocollagen structure prepared from the atelocollagen of the present invention and/or a drug delivery system including the solid atelocollagen structure show high viscosity and may be implanted in the body and easily adhered to the body. Further, the solid atelocollagen structure prepared from the atelocollagen of the present invention and/or a drug delivery system including the solid atelocollagen structure exhibit high viscosity and thus may be implanted in the body and may be easily bent. Moreover, it may be easily adhered to a curved region (e.g., a transplantation site in the body).

The drug delivery system including the atelocollagen of the present invention may reduce rapid initial release of the loaded physiologically active material or may regulate a decrease in effects of the physiologically active material due to too low initial release. The drug delivery system including the atelocollagen of the present invention may adjust a sustained release time of the loaded physiologically active material to the optimal range.

The drug delivery system prepared using the atelocollagen of the present invention may inhibit the loaded physiologically active material from being release too slowly, as compared to a drug delivery system prepared using the conventional atelocollagen, in which the area ($S_{\beta}$) of a peak for the $\beta$ chain is equal to or smaller than the area ($S_{\alpha 2}$) of a peak for the $\alpha 2$ chain, whereby efficacy of the physiologically active material may be enhanced. This may also be considered because a biodegradation rate may be regulated by increasing the content of the $\beta$ chain relative to the $\alpha 2$ chain of atelocollagen.

The physiologically active material may be a known material, and may be a low molecular weight compound, high molecular weight compound, aptamer, nucleic acid, nucleotide, a protein or polypeptide.

The physiologically active material may include an anticancer agent, an antibiotic, and the like, but it is not limited thereto. Further, any type of physiologically active material which is pharmaceutically acceptable may be used without limitation thereof. One type of physiologically active material may be loaded, or one or more types may be loaded.

Anticancer drugs may include alkylating agents such as Cyclophosphamide, Ifosfamide, Procarbazine, Busulfan, Carmustine, Lomustine, Dacarbazine, Cisplatin and the like. Anticancer drugs may include antimetabolites such as methotrexate, fluorouracil (5-fluorouracil), Capecitabine, Cytosine Arabinoside, Gemcitabine and the like. The anticancer agent may include a vinca alkaloid-based natural substance such as Vinblastine, vincristine and the like. The anticancer agent may include a taxane drug such as paclitaxel, docetaxel and the like. As the epipodophyllotoxin drug, etoposide and the like may be included. The anticancer agent may include a camptothecin drug such as Topotecan, Irinotecan and the like. The anticancer agent may include antitumor antibiotics such as mitomycin (Miitomycin-C), daunorubicin, doxorubicin, bleomycin and the like. The anticancer agent may include an enzyme-based drug such as asparaginase and the like. The anticancer agent may include a urea-based drug such as hydroxyurea and the like. As tyrosine kinase inhibitors, Rituximab, Trastuzumab, Imatinib, Gefitinib, Erlotinib, and the like may be included. The anticancer agent may include Opdivo, Keytruda, Yervoy, Tishentric, Kymria, Yescata, and the like as immune anticancer agents. Further, the anticancer agent may be in the form of a pharmaceutically acceptable salt or hydrate.

The drug delivery system of the present invention may be implanted in the body or injected into the body.

The drug delivery system of the present invention may be attached to, applied to, or inserted into a surgical site in an open state after surgery. According to an embodiment, the drug delivery system of the present invention may be attached to, applied to, or inserted into a cancer resection site in an open state after cancer surgery. According to an embodiment, the drug delivery system of the present invention may be attached, applied, or inserted to the remaining cancer site in an open state after cancer surgery.

The drug delivery system of the present invention may be subcutaneously implanted. The drug delivery system of the present invention may be adhered to or applied to the skin or mucous membrane. The drug delivery system of the present invention may be inserted into the oral cavity, the gastrointestinal tract or the like.

The drug delivery system of the present invention may be produced by any known method for manufacturing a drug delivery system.

According to an embodiment, the drug delivery system of the present invention may be prepared by a method including the following steps of:

adding 0.5 to 3% by weight ("wt.%") of atelocollagen of the present invention to a sodium acetate solution and stirring while maintaining pH 2 to 4; and
adding a buffer to the sodium acetate solution to which the atelocollagen was added (hereinafter, atelocollagen-sodium acetate solution) and substituting the sodium acetate solution with the buffer so as to obtain atelocollagen-buffer (hereinafter, atelocollagen dispersion).

The method for production of the drug delivery system may further include dispersing the physiologically active material in the atelocollagen dispersion obtained in the above-described step.

The buffer may be phosphate buffered saline (PBS).

In the step of obtaining the atelocollagen dispersion, the buffer may be added to the atelocollagen-sodium acetate solution using a tangential flow filtration system (Minimate™ TFF system, PALL), and the sodium acetate may be substituted with the buffer through a diafiltration process.

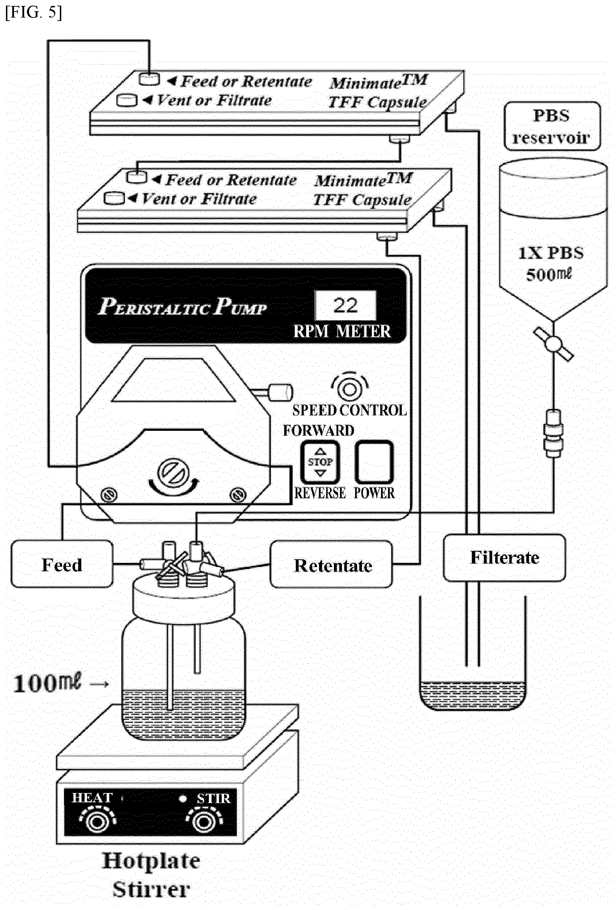

FIG. 5 is a schematic diagram illustrating a method of replacing atelocollagen-sodium acetate solution with atelocollagen-buffer by a tangential flow filtration device (Minimate TFF system).

The present invention provides a cancer metastasis inhibitor which includes the above-described atelocollagen.

The cancer metastasis inhibitor of the present invention may include the above-described atelocollagen and an anticancer agent loaded in the atelocollagen.

The anticancer agent may be a low molecular weight compound, high molecular compound, nucleic acid, peptide, protein or aptamer. The contents of the anticancer agent have been described above, and therefore will not be described in detail.

The cancer may be pancreatic cancer, breast cancer, liver cancer, head and neck cancer, colorectal cancer, ovarian cancer or glioblastoma.

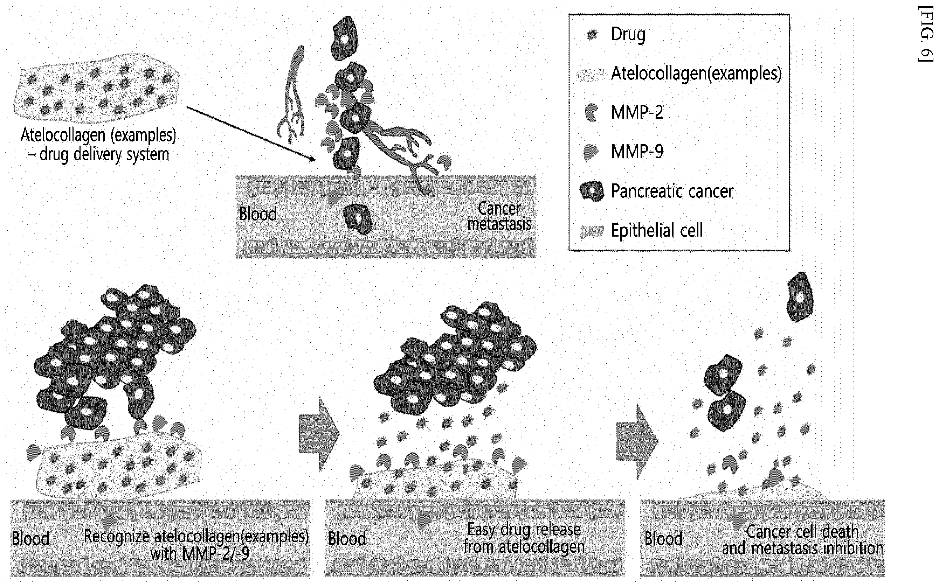

Cancer metastasis occurs when cancer cells infiltrate into tissues, it is known that the extracellular matrix and basement membrane are degraded by matrix metalloproteinase (MMP), an enzyme essential for protein degradation, in particular MMP-2 and MMP-9, resulting in infiltration of the cells (see the upper portion of FIG. 6).

As described above, when the atelocollagen of the present invention having a higher content of β chain than α2 chain is transplanted into cancer tissue, metastasis inhibitory effects appear. This may be considered because the atelocollagen of the present invention is recognized and degraded by MMP-2 and MMP-9, such that the extracellular matrix and a basement membrane present in the body of an individual with a cancer disease are relatively less degraded (see FIG. 6). Further, as the atelocollagen of the present invention is recognized and degraded by MMP-2 and MMP-9, a physiologically active material (e.g., an anticancer agent) loaded therein is released, resulting in the death of cancer cells.

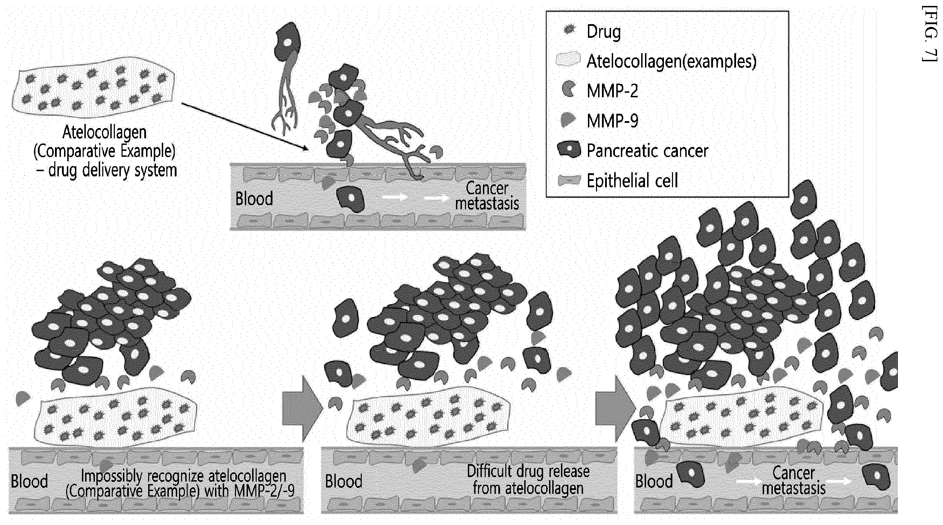

On the other hand, atelocollagen having a β chain content equal to or smaller than the α2 chain content (comparative examples) did not show metastasis inhibitory effects even when transplanted into cancer tissues. This may be considered because the atelocollagen having the β chain content equal to or smaller than the α2 chain content cannot be recognized and degraded by MMP-2 and MMP-9, instead, the extracellular matrix and the basement membrane present in the body of an individual with cancer are decomposed by the same (see FIG. 7).

Hereinafter, some examples will be given to describe the present invention in detail.

## 1. Preparation of atelocollagen (AC)

### 1) Example 1

A method of preparing atelocollagen in Example 1 will be described with reference to the flowchart of FIG. 4.

Step (1): Pig skin preparation and fragmentation step

Pig skin was washed 3 times with tap water, washed 3 times with primary purified water, and then divided into 2 kg (20 cm × 20 cm) and stored in a freezer at -20 °C. After standing at 4 °C for 2 hours and thawing, the frozen pig skin was cut into 1.5 cm × 8 cm size, 5 L of 0.5 M acetic acid was added, followed by leaving overnight to confirm swelling of the pig skin.

Step (2): Step of obtaining dermis

The swollen pig skin was taken out and the dermis was removed by means of a knife to remove the epidermal layer and the fat layer of the pig skin, and the tissue (dermis) from which the epidermal layer and fat layer were removed was cut into a size of 1.5 cm × 1.5 cm. Then, 5 L of fresh 0.5 M acetic acid was added to the dermis again and, after standing for several hours, only the dermis was collected by filtration using a sieve. The dermis was washed with 10 L of purified water. The washing process was repeated a total of 5 times. After adding 20 L of ethanol to the washed dermis, the mixture was stirred at 4 °C overnight. After completing the overnight stirring, only the dermis was recovered, 20 L of fresh ethanol was added, and the mixture was stirred again at 4 °C for 1 hour. Only the dermis was recovered using a sieve, and the ethanol was removed by standing still for about 1 hour. The dermis from which fat was removed was divided into appropriate weights (250 g) and stored in a freezer at -80 °C.

Step (3): Step of homogenizing dermis

After adding 0.625 L of 0.5 M acetic acid to the frozen dermis (250 g), it was thawed by standing for 30 minutes. At this time, the volume of 0.5 M acetic acid is preferably 0.25 L per 100 g of the dermis. Only the thawed dermis was recovered using a sieve and left still for about 1 hour to remove 0.5 M acetic acid. The following homogenization step was performed at 4 °C. 250 g of the dermis and 2L of tertiary distilled water were put into a blender and pulverized for 2 minutes, and then, 2L of purified water was further added and pulverized again for 2 minutes. 4 L of 0.5 M acetic acid was added to the pulverized tissue. At this time, the volume of 0.5 M acetic acid is preferably 4 L per 250 g of the dermis. Again, the tissue was homogenized for 3 minutes using a homogenizer. 4.5 L of 0.5 M acetic acid was further added to the homogenized tissue. While stirring using a stirrer, it was checked whether the pH was 2.5 or less and, when the pH is 2.5 or more, the pH was adjusted with a small amount of 4 M hydrochloric acid solution. It was stirred at a low speed for 3 hours using a stirrer.

Step (4): Step of treating homogenized dermal sample with pepsin

In the following pepsin treatment step, $1.5 \times 10^7$ unit of pepsin per 100 g of the dermis was added at 4 °C, and the dermal sample and the treated pepsin were stirred at a low speed overnight at 4 °C so that the pepsin was well mixed.

Step (5): Step of inactivating pepsin

The following pepsin inactivation step was performed at 4 °C. 10 M sodium hydroxide solution was added to the pepsin-treated sample and stirred to adjust the pH to 8 to 9, followed by further stirring for 10 minutes to inactivate pepsin.

Step (6): Step of adjusting pH

After pepsin inactivation by base treatment, a 4 M hydrochloric acid solution was added and stirred to adjust the pH to 3.4, followed by further stirring for 10 minutes.

Step (7) (including the following steps (7-1) and (7-2)): Collecting and salting out the supernatant of the pH-adjusted dermal sample

Step (7-1): The pH-adjusted sample was centrifuged at 7,800 rpm for 10 minutes at 4 °C using a centrifugal separator, and the remaining supernatant after removing the fat layer on the surface of the supernatant was collected. The supernatant from which the fat layer was removed was filtered once more through a sieve to collect only the supernatant.

Step (7-2): 5 M sodium chloride solution was slowly added to the finally obtained supernatant, stirred at 4 °C for 15 minutes, and then, left at 4 °C for 24 hours or overnight for salting out. At this time, the volume of the 5 M sodium chloride solution is preferably 16.3 ml per 100 g of the weight of the supernatant. After salting out, centrifugation was performed at 7,800 rpm for 10 minutes at 4 °C using a centrifuge. After centrifugation, the supernatant was removed to obtain a precipitate.

Step (8) (including the following steps (8-1) and (8-2)): Step of washing precipitate

Step (8-1): After adding 1 L of ethanol per 100 g to the precipitate obtained in the salting-out step, the mixture was first washed by stirring at 4 °C overnight. Then, centrifugation was performed at 7,800 rpm for 10 minutes at 4 °C using a centrifuge.

Step (8-2): After centrifugation, 1 L of ethanol per 100 g was added to the obtained precipitate, followed by second washing by stirring at 4 °C for 6 hours. The product was obtained after centrifugation at 7,800 rpm for 10 minutes at 4 °C using a centrifuge.

### 2) Example 2

A product was obtained in the same manner as in Example 1, but without performing steps (6) to (8).

### 3) Example 3

A product was obtained in the same manner as in Example 1, but without performing steps (7) to (8).

### 4) Example 4

A product was obtained in the same manner as in Example 1, but without performing step (8) (i.e., steps (8-1) and (8-2)).

### 5) Example 5

A product was obtained in the same manner as in Example 1, but without performing step (8-2).

### 6) Example 6

A product was obtained in the same manner as in Example 1 except that the step (4) (pepsin treatment step) was performed for 24 hours.

### 7) Example 7

A product was obtained in the same manner as in Example 1 except that the steps (6) to (8) were not performed, and the step (4) (pepsin treatment step) was performed for 24 hours.

### 8) Example 8

A product was obtained in the same manner as in Example 1 except that the steps (7) to (8) were not performed, and the step (4) (pepsin treatment step) was performed for 24 hours.

### 9) Example 9

A product was obtained in the same manner as in Example 1 except that step (8) was not performed, and step (4) (pepsin treatment step) was performed for 24 hours.

### 10) Example 10

A product was obtained in the same manner as in Example 1 except that step (8-2) was not performed, and step (4) (pepsin treatment step) was performed for 24 hours.

### 11) Example 11

A product was obtained in the same manner as in Example 1 except that steps (5) to (8) were not performed, and step (4) (pepsin treatment step) was performed for 1.5 hours.

### 12) Example 12

A product was obtained in the same manner as in Example 1 except that steps (5) to (8) were not performed, and the step (4) (pepsin treatment step) was performed for 3 hours.

### 13) Example 13

A product was obtained in the same manner as in Example 1 except that steps (5) to (8) were not performed, and the step (4) (pepsin treatment step) was performed for 4.5 hours.

### 14) Example 14

A product was obtained in the same manner as in Example 1 except that the steps (5) to (8) were not performed, and the step (4) (pepsin treatment step) was performed for 11.5 hours.

### 15) Example 15

A product was obtained in the same manner as in Example 1 except that steps (5) to (8) were not performed, and step (4) (pepsin treatment step) was performed at RT (room temperature) for 1.5 hours.

### 16) Example 16

A product was obtained in the same manner as in Example 1 except that the steps (6) to (8) were not performed, and the step (4) (pepsin treatment step) was performed for 1.5 hours.

### 17) Example 17

A product was prepared in the same manner as in Example 1 except that the steps (7) to (8) were not performed, and the step (4) (pepsin treatment step) was performed for 1.5 hours.

### 18) Example 18

A product was obtained in the same manner as in Example 1 except that step (8) was not performed, step (4) (pepsin treatment step) was performed for 1.5 hours, and the salting-out in step (7) was carried out for 17 hours.

### 19) Example 19

A product was obtained in the same manner as in Example 16 except that step (8-2) was not performed, and the salting-out in step (7) was carried out for 17 hours.

### 20) Example 20

A product was obtained in the same manner as in Example 1 except that step (4) (pepsin treatment step) was performed for 1.5 hours, and the salting-out was carried out for 17 hours.

**21) Example 21**

A product was obtained in the same manner as in Example 1 except that step (8) was not performed, step (4) (pepsin treatment step) was performed for 1.5 hours, and the salting-out in step (7) was carried out for 0.25 hours.

**22) Example 22**

A product was obtained in the same manner as in Example 1 except that step (8) was not performed, step (4) (pepsin treatment step) was performed for 1.5 hours, and the salting-out in step (7) was carried out for 1.5 hours.

**23) Example 23**

A product was obtained in the same manner as in Example 1 except that step (8) was not performed, step (4) (pepsin treatment step) was performed for 1.5 hours, and the salting-out in step (7) was carried out for 11.4 hours.

**24) Example 24**

A product was obtained in the same manner as in Example 1 except that step (8) was not performed, step (4) (pepsin treatment step) was performed for 1.5 hours, and the salting-out in step (7) was carried out for 14.2 hours.

**25) Example 25**

A product was obtained in the same manner as in Example 1 except that step (8) was not performed, step (4) (pepsin treatment step) was performed for 1.5 hours, and the salting-out in step (7) was carried out for 17 hours.

**26) Example 26**

A product was obtained in the same manner as in Example 1 except that steps (5) to (8) were not performed, and $0.75 \times 10^7$ unit of pepsin were added in step (4) (pepsin treatment step) while performing step (4) for 1.5 hours.

**27) Example 27**

A product was obtained in the same manner as in Example 1 except that steps (6) to (8) were not performed, and $0.75 \times 10^7$ unit of pepsin were added in step (4) (pepsin treatment step) while performing step (4) for 1.5 hours.

**28) Example 28**

A product was obtained in the same manner as in Example 1 except that steps (7) to (8) were not performed, and $0.75 \times 10^7$ unit of pepsin were added in step (4) (pepsin treatment step) while performing step (4) for 1.5 hours.

**29) Example 29**

A product was obtained in the same manner as in Example 1 except that step (8) was not performed, $0.75 \times 10^7$ unit of pepsin were added in step (4) (pepsin treatment step) while performing step (4) for 1.5 hours, and the salting-out in step (7) was carried out for 17 hours.

**30) Example 30**

A product was obtained in the same manner as in Example 1 except that step (8) was not performed, $0.75 \times 10^7$ unit of pepsin were added in step (4) (pepsin treatment step) while performing step (4) 1.5 hours, and the salting-out in step (7) was carried out for 17 hours.

**31) Example 31**

A product was obtained in the same manner as in Example 1 except that $0.75 \times 10^7$ unit of pepsin were added in step (4) (pepsin treatment step) while performing step (4) for 1.5 hours, and the salting-out was carried out for 17 hours.

**32) Example 32**

A product was obtained in the same manner as in Example 1 except that step (8) was not performed, $0.75 \times 10^7$ unit of pepsin were added in step (4) (pepsin treatment step) while performing step (4) for 1.5 hours, and the salting-out in step (7) was carried out for 0.25 hours.

**33) Example 33**

A product was obtained in the same manner as in Example 1 except that step (8) was not performed, $0.75 \times 10^7$ unit of pepsin were added in step (4) (pepsin treatment step) while performing step (4) for 1.5 hours, and the salting-out in step (7) was carried out for 1.5 hours.

**34) Example 34**

A product was obtained in the same manner as in Example 1 except that step (8) was not performed, $0.75 \times 10^7$ unit of pepsin were added in step (4) (pepsin treatment step) while performing step (4) for 1.5 hours, and the salting-out in step (7) was carried out for 11.4 hours.

**35) Example 35**

A product was obtained in the same manner as in Example 1 except that step (8) was not performed, $0.75 \times 10^7$ unit of pepsin were added in step (4) (pepsin treatment step) while performing step (4) for 1.5 hours, and the salting-out in step (7) was carried out for 14.2 hours.

**36) Example 36**

A product was obtained in the same manner as in Example 1 except that step (8) was not performed, $0.75 \times 10^7$ unit of pepsin were added in step (4) (pepsin treatment step) while performing step (4) for 1.5 hours, and the salting-out in step (7) was carried out for 17 hours.

**37) Example 37**

A product was obtained in the same manner as in Example 1 except that steps (5) to (8) were not performed, and step (4) (pepsin treatment step) was performed at 4 °C for 1.5 hours and RT (room temperature) for 2.68 hours.

**38) Example 38**

A product was obtained in the same manner as in Example 1 except that steps (6) to (8) were not performed, and step (4) (pepsin treatment step) was performed at 4 °C for 1.5 hours and RT (room temperature) for 2.68 hours.

**39) Example 39**

A product was obtained in the same manner as in Example 1 except that steps (7) to (8) were not performed, and step (4) (pepsin treatment step) was performed at 4 °C for 1.5 hours and RT (room temperature) for 2.68 hours.

**40) Example 40**

A product was obtained in the same manner as in Example 1 except that step (8) was not performed, step (4) (pepsin treatment step) was performed at 4 °C for 1.5 hours and RT (room temperature) for 2.68 hours, and the salting-out in step (7) was carried out for 17 hours.

**41) Example 41**

A product was obtained in the same manner as in Example 1 except that step (8-2) was not performed, step (4) (pepsin treatment step) was performed at 4 °C for 1.5 hours and RT (room temperature) for 2.68 hours, and the salting-out in step (7) was carried out for 17 hours.

**42) Example 42**

A product was obtained in the same manner as in Example 1 except that step (4) (pepsin treatment step) was performed at 4 °C for 1.5 hours and RT (room temperature) for 2.68 hours, and the salting-out was carried out for 17 hours.

**43) Example 43**

A product was obtained in the same manner as in Example 1 except that steps (5) to (8) were not performed, and step (4) (pepsin treatment step) was performed at 4 °C for 8 hours.

**44) Example 44**

A product was obtained in the same manner as in Example 1 except that steps (5) to (8) were not performed, and step (4) (pepsin treatment step) was performed at 4° C for 8 hours and RT (room temperature) for 2.68 hours.

**45) Example 45**

A product was obtained in the same manner as in Example 1 except that step (7-2) and step (8) were not performed, and step (4) (pepsin treatment step) was performed at 4° C for 1.5 hours and RT (room temperature) for 2.68 hours.

**46) Example 46**

A product was obtained in the same manner as in Example 1, except that step (8) was not performed, step (4) (pepsin treatment step) was performed at 4° C for 1.5 hours and RT (room temperature) for 2.68 hours, and the salting-out in step (7) was carried out for 5 hours.

**47) Example 47**

A product was obtained in the same manner as in Example 1 except that step (8) was not performed, step (4) (pepsin treatment step) was performed at 4 °C for 1.5 hours and RT (room temperature) for 2.68 hours, and the salting-out in step (7) was carried out for 10 hours.

**48) Example 48**

A product was obtained in the same manner as in Example 1, except that step (8) was not performed, step (4) (pepsin treatment step) was performed at 4 °C for 1.5 hours and RT (room temperature) for 2.68 hours, and the salting-out in step (7) was carried out for 12.5 hours.

**49) Example 49**

A product was obtained in the same manner as in Example 1 except that step (8) was not performed, step (4) (pepsin treatment step) was performed at 4 °C for 1.5 hours and RT (room temperature) for 2.68 hours, and the salting-out in step (7) was carried out for 14 hours.

**50) Example 50**

A product was obtained in the same manner as in Example 1, except that step (8) was not performed, and step (4) (pepsin treatment step) was performed at 4 °C for 1.5 hours and RT (room temperature) for 2.68 hours, and the salting-out in step (7) was carried out for 15 hours.

**51) Example 51**

A product was obtained in the same manner as in Example 1 except that step (8) was not performed, step (4) (pepsin treatment step) was performed at 4 °C for 1.5 hours and RT (room temperature) for 2.68 hours, and the salting-out in step (7) was carried out for 17 hours.

**52) Comparative Example 1**

Commercially available atelocollagen was prepared in Comparative Example 1 (Matrixen-PSP, SK Bioland).

**53) Comparative Example 2**

Commercially available atelocollagen was prepared in Comparative Example 2 (COLTIX® Tendoregen, Eubiosis).

**54) Comparative Example 3**

A product was obtained in the same manner as in Example 1, but without performing steps (5) to (8).

**55) Comparative Example 4**

A product was obtained in the same manner as in Example 1 except that steps (5) to (8) were not performed, and step (4) (pepsin treatment step) was performed for 24 hours.

**56) Comparative Example 5**

A product was obtained in the same manner as in Example 1 except that steps (5) to (8) were not performed, and step (4) (pepsin treatment step) was performed for 13 hours.

**57) Comparative Example 6**

A product was obtained in the same manner as in Example 1 except that steps (5) to (8) were not performed, step (4) (pepsin treatment step) was performed at RT (room temperature) for 20 hours.

**58) Comparative Example 7**

A product was obtained in the same manner as in Example 1 except that steps (6) to (8) were not performed, step (4) (pepsin treatment step) was performed at RT (room temperature) for 20 hours.

**59) Comparative Example 8**

A product was obtained in the same manner as in Example 1 except that steps (7) to (8) were not performed, and step (4) (pepsin treatment step) was performed at RT (Room temperature) for 20 hours.

**60) Comparative Example 9**

A product was obtained in the same manner as in Example 1 except that step (8) was not performed, and step (4) (pepsin treatment step) was performed at RT (room temperature) for 20 hours.

**61) Comparative Example 10**

A product was obtained in the same manner as in Example 1 except that step (8-2) was not performed, and step (4) (pepsin treatment step) was performed at RT (room temperature) for 20 hours.

**62) Comparative Example 11**

A product was obtained in the same manner as in Example 1 except that (4) step (pepsin treatment step) was performed at RT (room temperature) for 20 hours.

**63) Comparative Example 12**

A product was obtained in the same manner as in Example 1 except that steps (5) to (8) were not performed, and step (4) (pepsin treatment step) was performed at RT (room temperature) for 3 hours.

**64) Comparative Example 13**

A product was obtained in the same manner as in Example 1 except that steps (5) to (8) were not performed, and step (4) (pepsin treatment step) was performed at RT (room temperature) for 4.5 hours.

**65) Comparative Example 14**

A product was obtained in the same manner as in Example 1 except that steps (5) to (8) were not performed, and step (4) (pepsin treatment step) was performed at RT (room temperature) for 11.5 hours.

**66) Comparative Example 15**

A product was obtained in the same manner as in Example 1 except that steps (5) to (8) were not performed, and step (4) (pepsin treatment step) was performed at RT (room temperature) for 13 hours.

**2. Preparation of atelocollagen solution with neutral pH**

**(1)** Production of high-purity atelocollagen

A 0.02 M urea solution was added to each of the products prepared in the examples and comparative examples in "1. Preparation of atelocollagen (AC)" above, followed by stirring the same at 4 °C overnight. At this time, the volume of the 0.02 M urea solution is preferably 1.4 L per 100 g of atelocollagen. Ultrafiltration and diafiltration of the atelocollagen solution using a Centramate Tangential Flow Filtration (TFF) System or a Minimate tangential flow filtration system (Minimate TFF system) was conducted to obtain high-purity atelocollagen. A 0.5 M sodium hydroxide solution was added to the obtained atelocollagen solution while stirring so as to reach pH 7. The prepared neutral atelocollagen was thinly and flatly subdivided in a zipper bag, and stored in a -80 °C ultra-low temperature ("cryogenic") freezer. After pre-freezing (-40 °C) in the lyophilizer for at least 1 hour, the atelocollagen stored in the -80 °C cryogenic freezer was put into the lyophilizer and freeze-dried (i.e., lyophilized). After the lyophilization was completed, the atelocollagen was cut into appropriate sizes, vacuum-packed, and then refrigerated at an appropriate temperature.

**(2) Preparation of atelocollagen solution with neutral pH**

By dissolving 2.4 g of sodium acetate ($CH_3CO_2Na$) in 55 ml of tertiary sterile water and 45 ml of an acetic acid solution with a purity of 99% or higher, a sodium acetate solution was prepared (0.3 M sodium acetate, 45% acetic acid). Thereafter, using a pH meter, the sodium acetate solution (0.3 M sodium acetate, 45% acetic acid) was titrated to reach pH 3.0. While stirring the sodium acetate solution using a stirrer, each of the high-purity products prepared in the examples and comparative examples of the above "2. (1) Production of high-purity atelocollagen" was added in an amount of 0.5 to 3 wt.% and stirred overnight. As a result, atelocollagen-sodium acetate solution was obtained.

Diafiltration using a Minimate™ Tangential Flow Filter system (PALL) was conducted to substitute the atelocollagen-sodium acetate solution with atelocollagen-phosphate buffered saline (PBS) solution. Specifically, a tangential flow filtration system (Minimate™ Tangential Flow Filter system) was prepared by mounting a membrane (Minimate TFF capsule, PALL) on a continuous metering pump (Peristaltic pump, Poongrim). In this case, the pores of the membrane preferably have a size of 100 kDa. As a reservoir for the atelocollagen-sodium acetate solution and the phosphate buffered saline (PBS reservoir) is connected to the prepared system (see FIG. 5), whereby the sodium acetate solution passes through a filtrate tube while the remaining solution moves through a retentate tube. Difiltration was performed by adding 1 × PBS solution to the PBS reservoir in order to keep a level of the atelocollagen solution constant. Diafiltration was continued using 1 × PBS solution in a volume corresponding to 10 times the atelocollagen-sodium acetate solution supplied initially and, as a result, buffer exchange was executed. After the buffer exchange was completed, it could be confirmed that the pH of the solution passing through the filtrate tube was neutral pH. The pH neutral atelocollagen solution after buffer exchange was recovered and refrigerated at 4 °C.

**(3) Confirmation of purity of atelocollagen in atelocollagen solution with neutral pH**

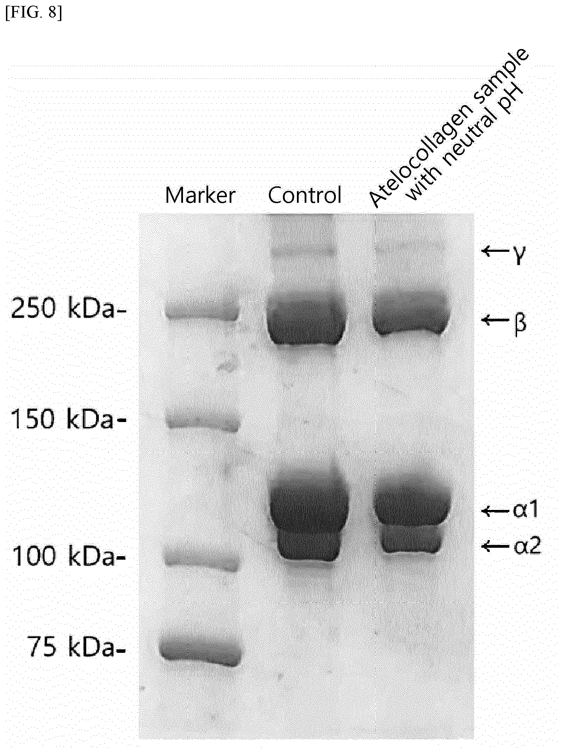

Using SDS-PAGE electrophoresis, the purity of atelocollagen in the atelocollagen solution prepared in the above "2. (2) Preparation of atelocollagen solution with neutral pH" above was confirmed. Specifically, the product prepared in Example 1 of the above "2. (1) Production of high-purity atelocollagen" was dissolved in 0.5 M acetic acid solution at 0.3 wt.% and used as a control. The atelocollagen solution (the atelocollagen sample with neutral pH) prepared in the above "2. (2) Preparation of atelocollagen solution with neutral pH" was diluted 10-fold in 0.5 M acetic acid solution to prepare an atelocollagen analysis sample. 5X sample buffer was mixed with the sample to become 1X, and heated at 85 °C for 5 minutes. Samples prepared on 6% SDS-PAGE gel were loaded and electrophoresed. Electrophoresis was conducted at 90 V for 30 minutes, and then the voltage was increased to 120 V and developed until 75 kDa of the size of the gel marker came down to the bottom of the gel. When the sample was well separated on the gel, the gel was taken out and stained with Coomassie Brilliant Blue R250 Staining Solution (BIO-RAD) at room temperature for 2 hours or at 4 °C for 16 hours. The gel stained on the rocker was washed with a de-staining solution so that the atelocollagen band was clearly visible. Through the above analysis method, it was possible to confirm the purity of the atelocollagen sample prepared in the above 2. (2) (see FIG. 8).

### 3. Comparison of chain content of atelocollagen through high-performance liquid chromatography (HPLC) analysis

#### (1) Confirmation of distribution of each chain of atelocollagen in HPLC analysis

Using a high-performance liquid chromatography (HPLC) instrument, atelocollagen samples were prepped, and then SDS-PAGE analysis was performed to confirm the atelocollagen chain distribution in HPLC analysis.

A 0.5 M acetic acid solution containing atelocollagen was prepared at a concentration of 5.0 $\mu g/\mu l$ and analyzed using an Agilent HPLC instrument and a UV detector (220 nm) as a detector. The column was set to gradient using a reverse phase column RP C18 column, a mobile phase including Buffer A: 0.1 % TFA in DW and Buffer B: 0.1 % TFA in ACN, and a flow rate of 1 ml/min. First, 30 $\mu l$ of the atelocollagen sample was injected to confirm four atelocollagen peaks in the chromatogram. 100 $\mu l$ of the atelocollagen sample was injected and only the peak having a retention time of 11 to 14.5 minutes in the chromatogram was fractionated, and then the fraction was reanalyzed to confirm the degree of peak separation. Of the fractions for which the degree of separation was confirmed, only the atelocollagen main peaks were collected and analyzed by SDS-PAGE for each peak.

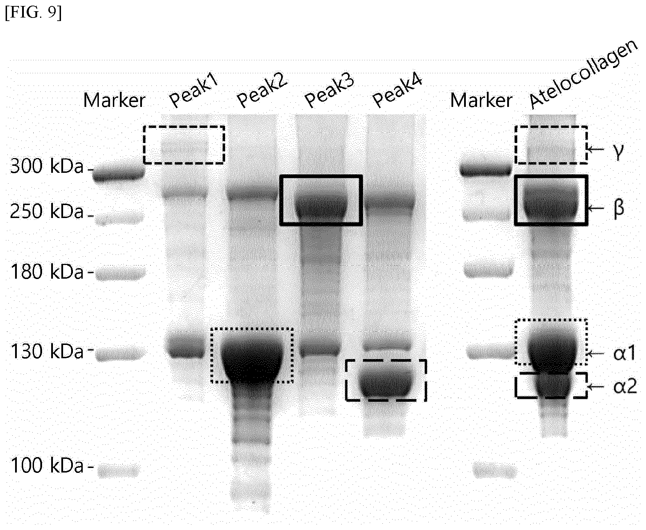

As a result of the analysis, it could be confirmed that Peak 1 with the smallest elution time is a peak for the $\gamma$ chain, the second smallest Peak 2 is a peak for the $\alpha1$ chain, the third smallest Peak 3 is a peak for the $\beta$ chain, and the largest Peak 4 is a peak for the $\alpha2$ chain (see FIG. 9).

#### (2) Comparison of chain content of atelocollagen through HPLC analysis

The analysis sample was prepared at a concentration of 2.5 $\mu g/\mu l$ by dissolving each of the high-purity products in the examples and comparative examples of the above "2. (1) Production of high-purity atelocollagen" in 0.5 M acetic acid solution.

Among analysis conditions, an Agilent HPLC instrument was used and a UV detector (220 nm) was used as a detector. The column was set to gradient using a reverse phase column RP C18 column and a mobile phase including Buffer A: 0.1 % TFA in D.W and Buffer B: 0.1 % TFA in ACN, and a flow rate thereof was 1 ml/min.

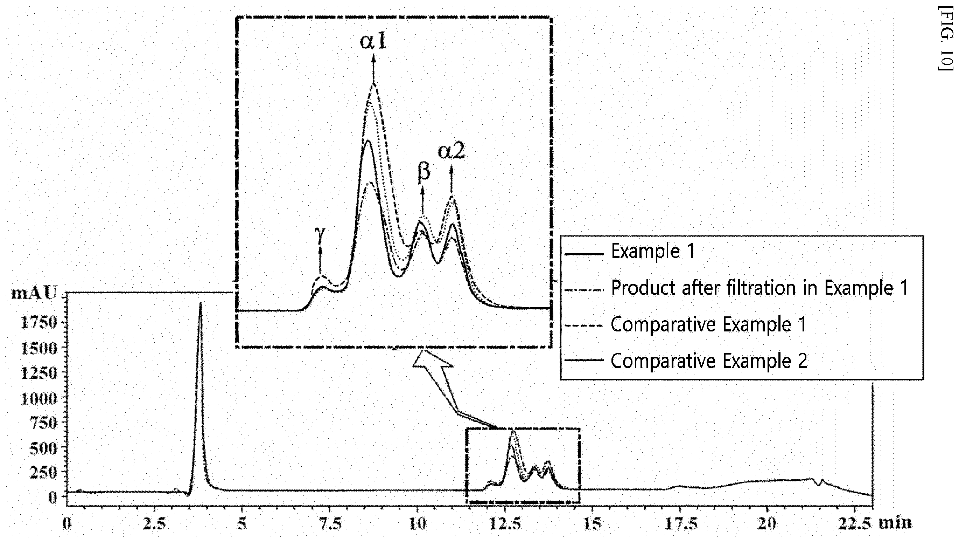

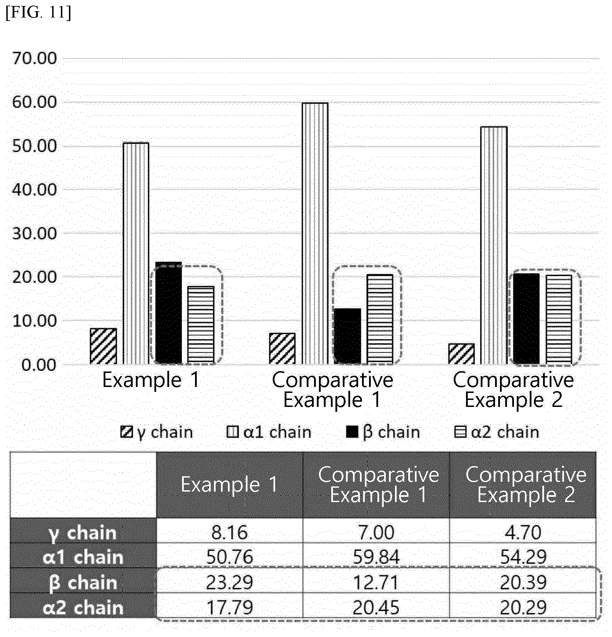

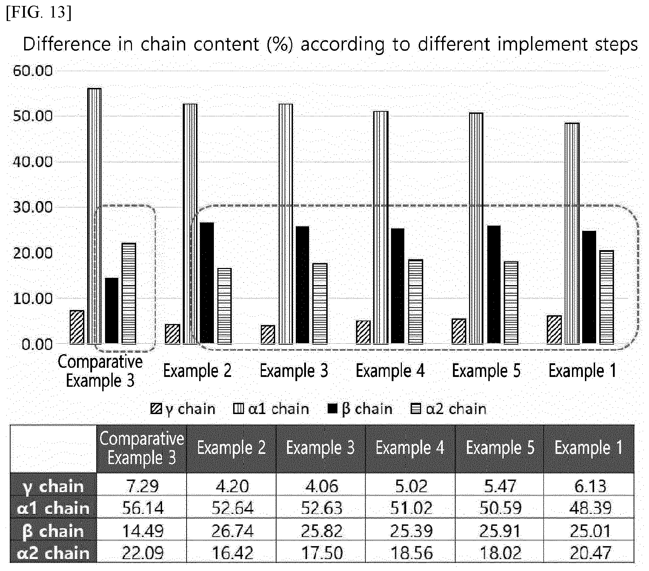

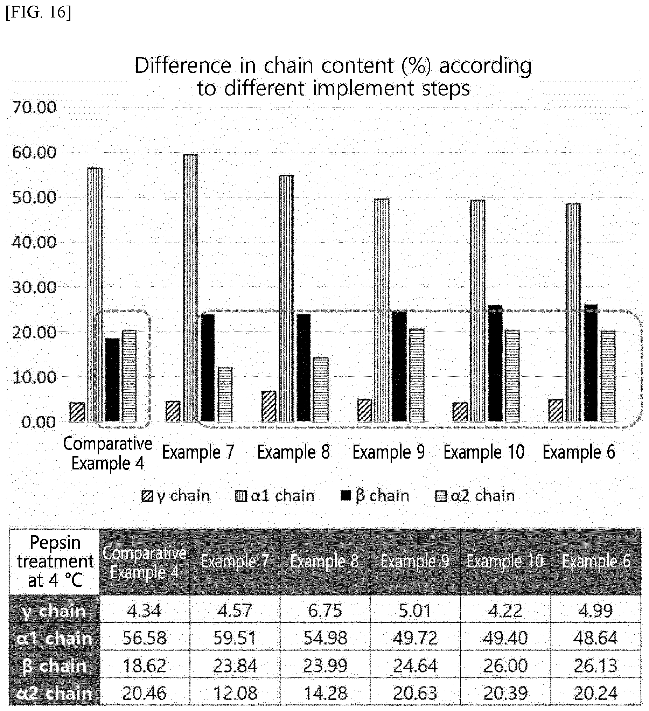

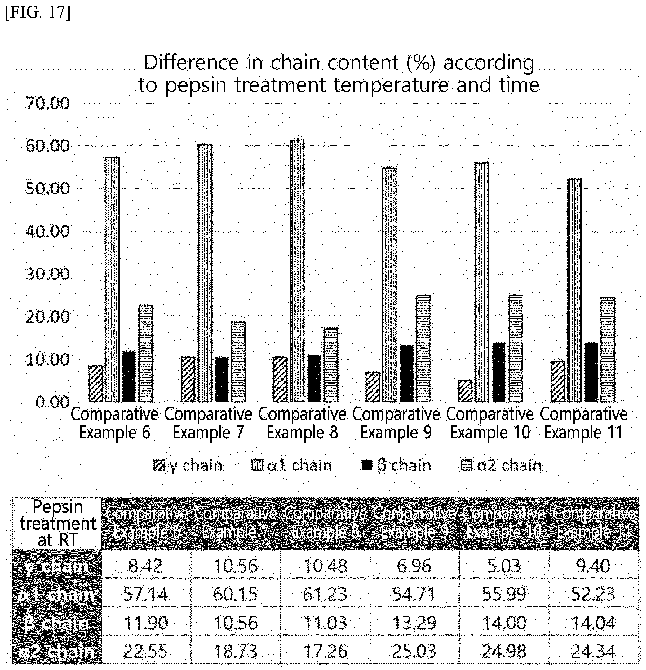

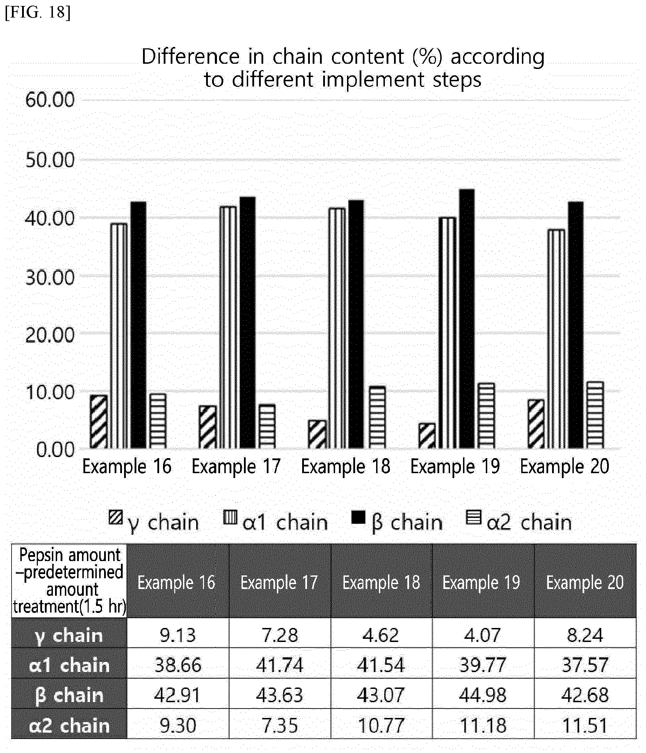

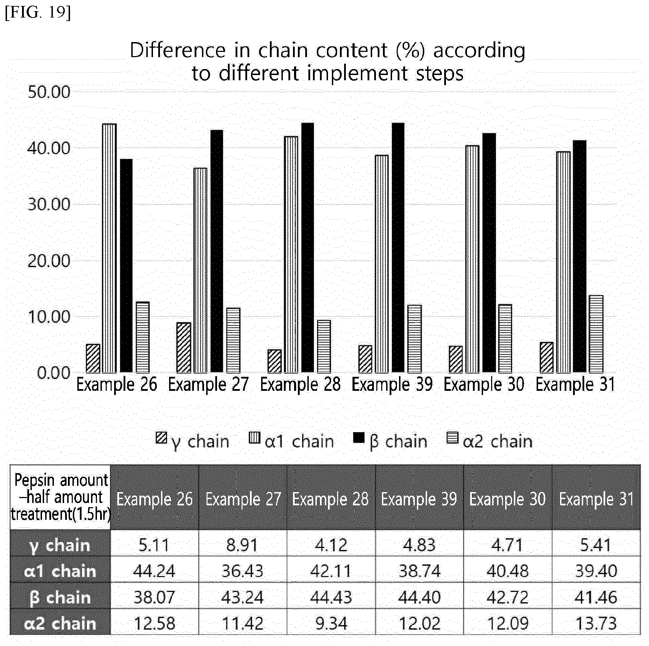

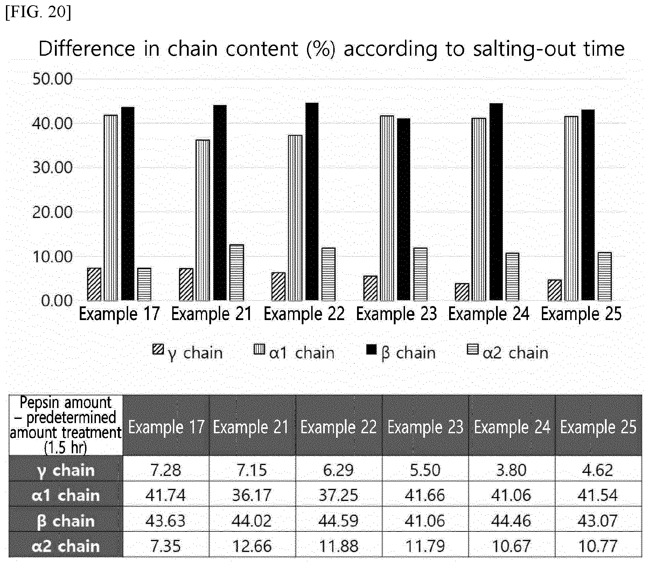

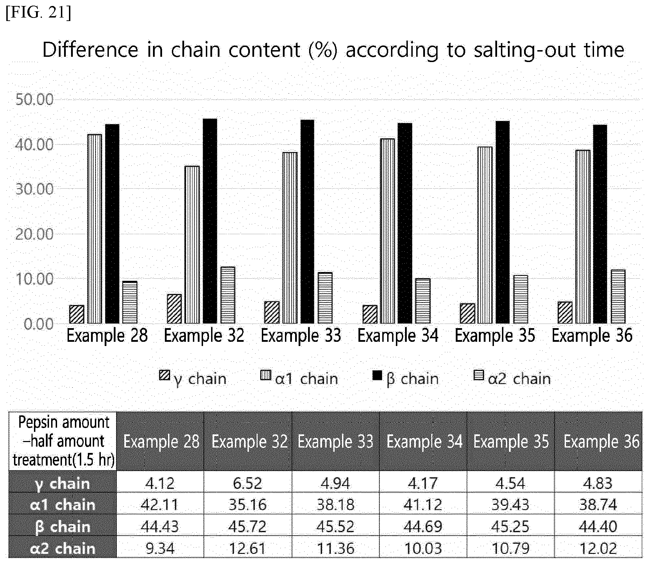

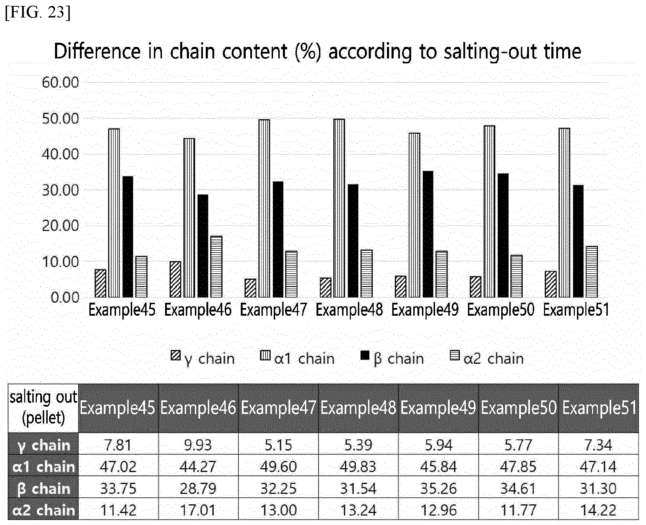

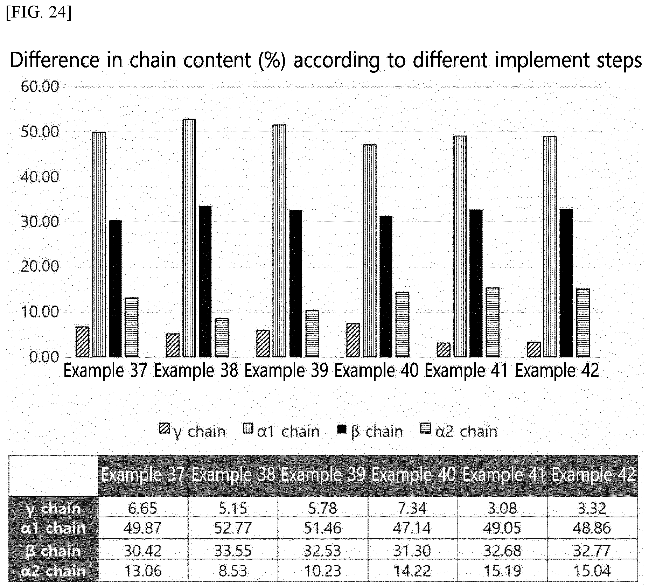

As a result of measuring the area under the ridge including the peak corresponding to each chain by high-performance liquid chromatography analysis of examples and comparative examples, Examples 1 to 51 showed that the area of the peak for the $\beta$ chain ($S_\beta$) is larger than the area of the peak for the $\alpha2$ chain ($S_{\alpha2}$). On the other hand, in Comparative Examples 1 to 15, it was confirmed that the area of the peak for the $\beta$ chain ($S_\beta$) was equal to or smaller than the area of the peak for the $\alpha2$ chain ($S_{\alpha2}$) (see FIGS. 10 to 24). That is, the atelocollagen of the present invention has a higher content of $\beta$ chain than $\alpha2$ chain, unlike other existing atelocollagen. This difference in chain contents may affect physical properties and effects of the atelocollagen of the present invention.

More specifically, with regard to the commercially available atelocollagen (Comparative Examples 1 and 2), the $\beta$ chain content is almost equal to or smaller than the $\alpha2$ chain content, whereas the atelocollagen of the present invention has a larger $\beta$ chain content compared to the $\alpha2$ chain, wherein the content of the $\beta$ chain relative to the $\alpha2$ chain was 1.1 or more and 5 or less (see FIGS. 10 to 11).

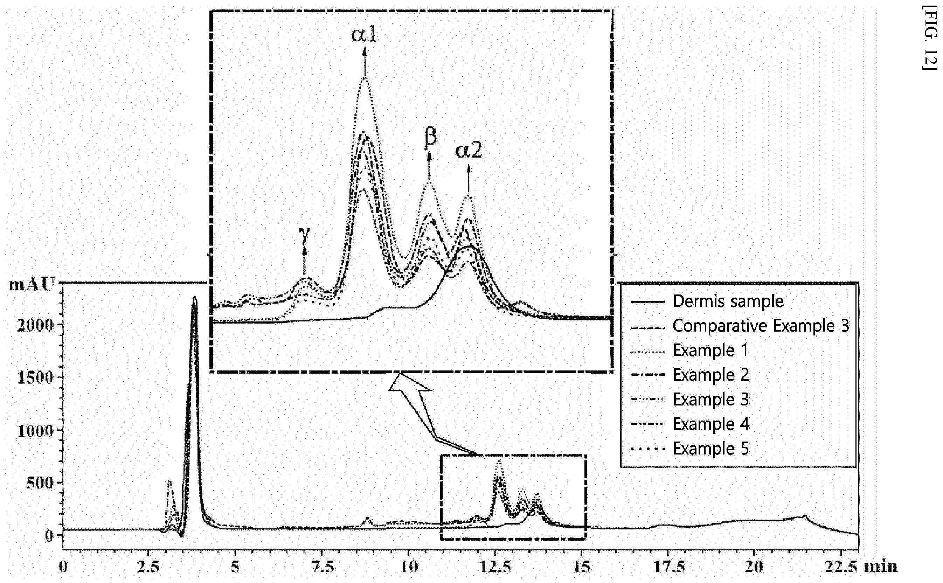

Further, it was confirmed that the atelocollagen produced by performing the step of inactivating pepsin (step (5) described in Example 1) had a greater content of $\beta$ chains relative to $\alpha2$ chains than the other cases (FIGS. 12, 13, 16, 19 and 24).

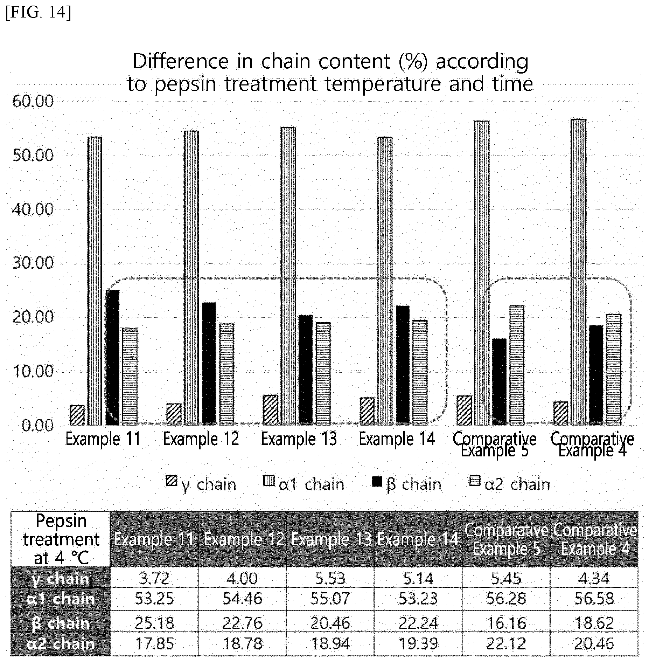

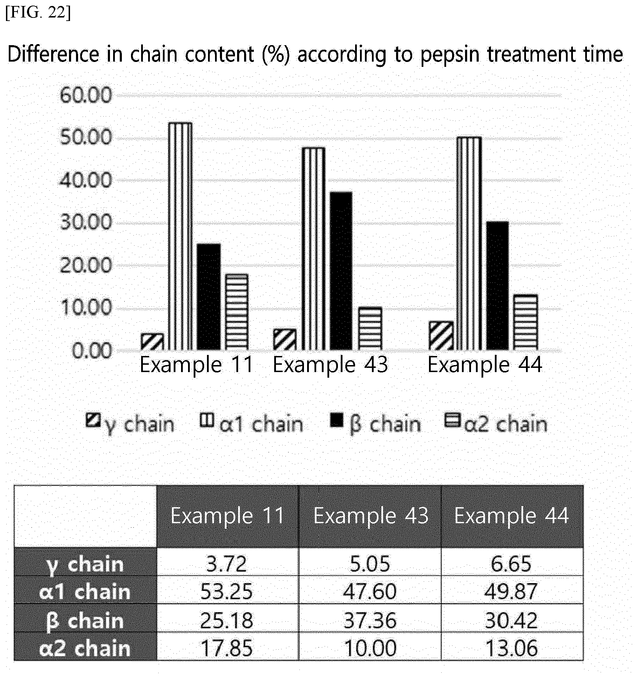

Even if the step of inactivating pepsin (step (5) described in Example 1) is not performed, when the pepsin treatment step (step (4) described in Example 1) is performed at 4 °C for less than 13 hours, it could be confirmed that the content of the $\beta$ chain relative to the $\alpha2$ chain was larger. On the other hand, when the pepsin treatment step was performed for 13 hours or more, the content of the $\alpha2$ chain was greater than that of the $\beta$ chain (see FIGS. 14 and 22).

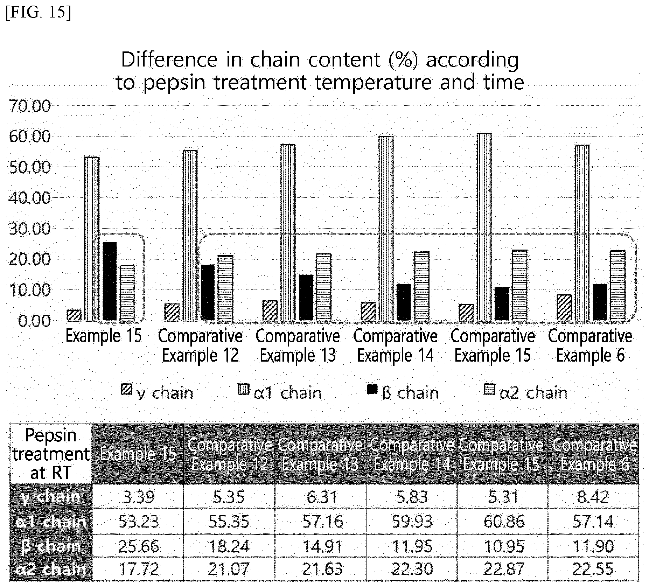

Even if the step of inactivating pepsin (step (5) described in Example 1) is not performed, when the pepsin treatment step (step (4) described in Example 1) is performed at RT for less than 3 hours, it could be confirmed that the content of the $\beta$ chain relative to the $\alpha2$ chain was increased. On the other hand, when the pepsin treatment step was performed at RT for 3 hours or more, the content of the $\alpha2$ chain was higher than that of the $\beta$ chain (see FIGS. 15, 17 and 19).

Further, each chain content as well as a ratio of β chain: α2 chain in the atelocollagen (examples and comparative examples) obtained by performing all of steps (1) to (8) in the above examples and comparative examples are shown in Table 2 below.

[TABLE 2]

| Section | | Comparative Example 11 | Example 1 | Example 42 | Example 31 | Example 20 |
|---|---|---|---|---|---|---|
| Conditions of pepsin treatment | Temperature and time | RT, 20 hours | 4 °C, Overnight | 4 °C, 8 hours + RT, 2.68 hours | 4 °C, 1.5 hours | 4 °C, 1.5 hours |
| | Amount of pepsin | Based on 100 g of dermis, $1.5 \times 10^7$ unit | Based on 100 g of dermis, $1.5 \times 10^7$ unit | Based on 100 g of dermis, $1.5 \times 10^7$ unit | Based on 100 g of dermis, $0.75 \times 10^7$ unit | Based on 100 g of dermis, $1.5 \times 10^7$ unit |
| γ chain | | 9.40 | 6.13 | 3.32 | 5.41 | 8.24 |
| α1 chain | | 52.23 | 48.39 | 48.86 | 39.40 | 37.57 |
| β chain | | 14.04 | 25.01 | 32.77 | 41.46 | 42.68 |
| α2 chain | | 24.34 | 20.47 | 15.04 | 13.73 | 11.51 |
| β chain: α2 chain (ratio) | | 0.58:1 | 1.22:1 | 2.18:1 | 3.02:1 | 3.69:1 |

According to the above analysis results, i) inactivating pepsin during the production of atelocollagen (step (5) described in Example 1) and/or adjusting the pH after inactivating pepsin (described in Example 1 (6) step), or ii) treating the dermis with pepsin (step (4) described in Example 1) under specific conditions including a specific temperature and reaction time (e.g., at 4 °C for less than 13 hours, or RT (room temperature) for less than 3 hours) may enable production of atelocollagen having a higher content of β chains than α2 chains. According to the above results, it was confirmed that the ratio of β chains:α2 chains in the atelocollagen could be adjusted depending on whether specific steps were performed in the production process of atelocollagen or pepsin treatment conditions (pepsin treatment time, pepsin treatment temperature, etc.).

## 4. Comparison of chain content of atelocollagen through SDS-PAGE analysis

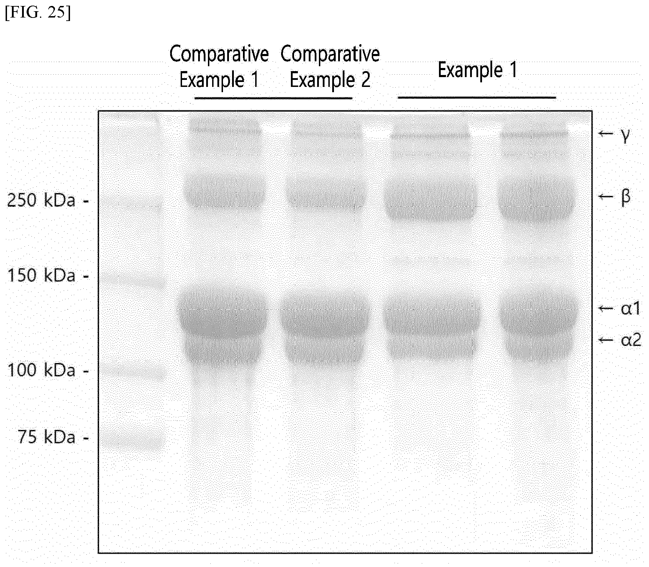

Sodium dodecyl sulfate polyacrylamide gel electrophoresis (SDS-PAGE) analysis was performed in the same manner as in "2. (3) Confirmation of purity of atelocollagen in atelocollagen solution with neutral pH" above. As a result of measuring a relative gel band intensity of the gel image obtained from the above analysis using ImageJ program, it was confirmed that a band thickness for the β chain of Example 1 was significantly thicker than a band thickness for the β chain of Comparative Example 1 or 2. That is, it was confirmed that the β chain content of the atelocollagen of the example was greater than that of the comparative example (see FIG. 25).

## 5. Assessment of physical properties of atelocollagen

### (1) Formation of solid atelocollagen structure

The high-purity atelocollagen prepared in each of the examples and comparative examples was poured into a silicone mold and lyophilized to form a solid atelocollagen structure.

Specifically, a predetermined amount of the high-purity atelocollagen solution was dispensed into a silicone mold. The silicone mold in which the atelocollagen solution was dispensed was first frozen at -20 °C for 4 hours or more. After the first freezing was completed, the sample was separated from the silicone mold and transferred to a sterile dish or plate, and then frozen at -80 °C for 2 hours or more. After pre-freezing so that a cold trap temperature of a lyophilizer reaches -80 °C, the atelocollagen sample was put into a lyophilizer and lyophilized for at least 16 hours. The lyophilized solid atelocollagen structure was refrigerated at 4 °C.

**(2) Observation of structure of atelocollagen through scanning electron microscope (SEM)**

Using a scanning electron microscope (SEM), it was confirmed whether the structure of atelocollagen was changed according to a ratio of $\beta$ chain and $\alpha2$ chain of atelocollagen. Specifically, the atelocollagen solution in each of Example 1, Example 42, Example 31, Comparative Example 1 and Comparative Example 2 was lyophilized to prepare a circular sample having a diameter of 10 mm and a height of 5 mm. After coating the surface of the prepared sample using a platinum coating device, the coated sample was put into an SEM chamber, followed by applying a high voltage of 10.0 to 15.0 kV to the sample and then measuring the surface, cross side and bottom of the sample at magnifications of 50X, 500X and 1000X. Thereafter, pore diameters of the prepared samples at the same magnification were measured, and the average diameter and standard deviation of the pores were calculated.

As a result of the measurement, it was confirmed that all the atelocollagen samples of Examples 1, 42, and 31 had pores open on both sides, and the average diameter of the pores was decreased as the content of $\beta$ chain relative to $\alpha2$ chain was increased (see FIG. 26 and Table 3). On the other hand, Comparative Example 1 contained pores closed on one side and Comparative Example 2 contained pores with one wide side and the other narrow side, while the average diameter of the pores of Comparative Examples 1 and 2 was significantly larger than that of the examples. These results suggest that physical properties may vary depending on the content ratio of $\beta$ chain to $\alpha2$ chain.

[TABLE 3]

| Section | Example 1 | Example 42 | Example 31 | Comparative Example 1 | Comparative Example 2 |
|---|---|---|---|---|---|
| $\beta$ chain: $\alpha2$ chain | 1.22:1 | 2.18:1 | 3.02:1 | 0.62:1 | 1.00:1 |
| Average diameter of pore | 72.8um | 62.5um | 35.6um | 119.23um | Wide side: 120.78 $\mu$m<br>Narrow side: 46.89 $\mu$m |
| Standard deviation of pore diameter | 22.1um | 17.0um | 2.2um | 22. Hum | Wide side: 10.42 $\mu$m<br>Narrow side: 5.75 $\mu$m |
| Pore type | Pore open on both sides | | | Pore closed on one side | Pore with one wide side and the other narrow side |

**(3) Measurement of tensile strength of atelocollagen**

It was investigated whether the strength of atelocollagen was changed according to the ratio of $\beta$ and $\alpha2$ chains of atelocollagen.

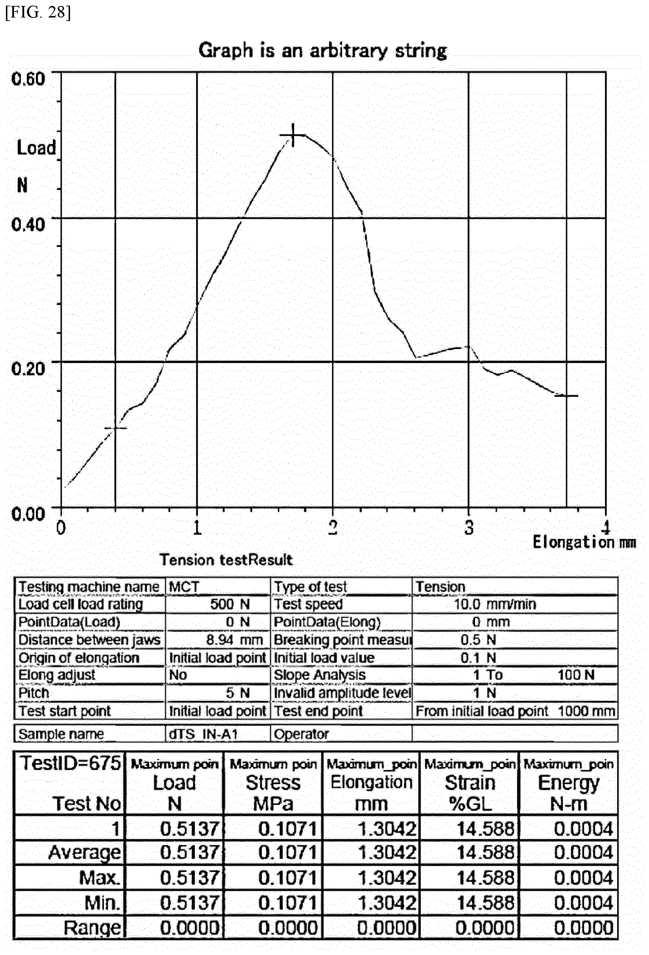

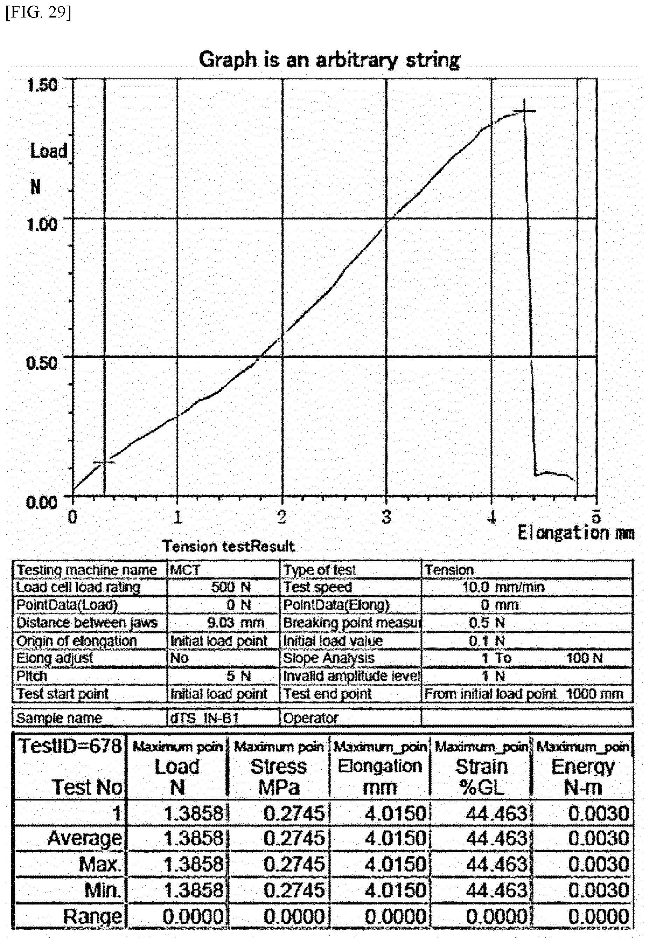

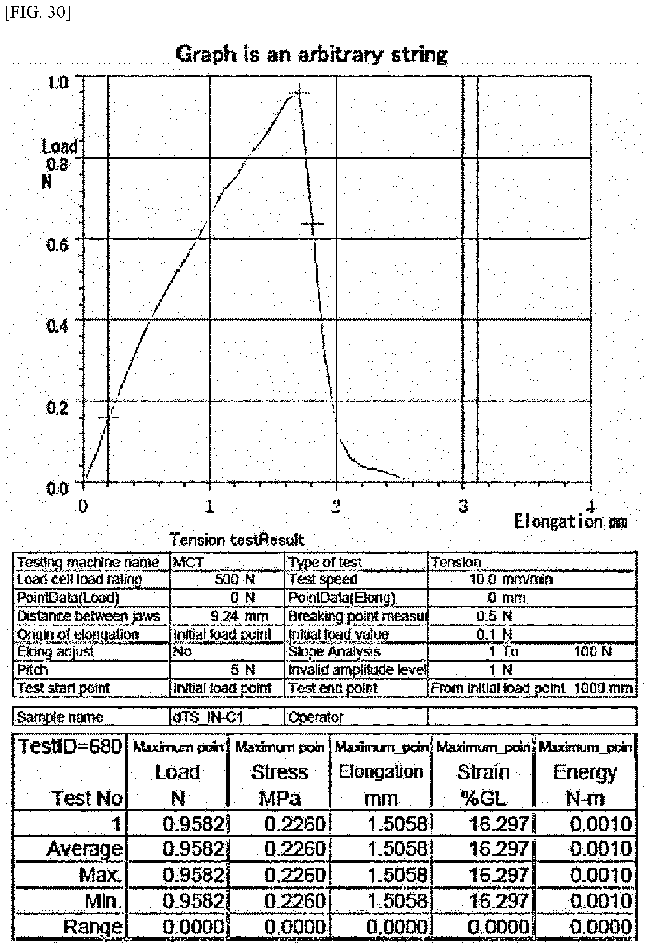

The atelocollagen solutions of Examples 1 and 31 and the atelocollagen solution of Comparative Example 2 were lyophilized to prepare each test material for tensile strength test with a size of 20 mm $\times$ 3 mm $\times$ 2 mm. Using a tensile compression tester (MCT-1150), tension was uniformly applied at a speed of 10.0 mm/min in the longitudinal axis direction of the test material to measure the maximum tension until the material is broken, thereby calculating the tensile strength (maximum stress) and tensile strain (maximum tensile strain). It was observed significant differences in the tensile strength and tensile strain values between the examples, in which the $\beta$ chain content was greater than the $\alpha2$ chain content, and the comparative example, in which the $\beta$ chain content was smaller than the $\alpha2$ chain content (see Table 4, and FIGS. 28 to 30).

[TABLE 4]

| Section | Example 1 | Example 31 | Comparative Example 2 |
|---|---|---|---|
| $\beta$ chain : $\alpha2$ chain | 1.22:1 | 3.02:1 | 1.00:1 |
| Tensile strength (maximum tensile stress) [Mpa] | 0.107 | 0.226 | 0.305 |
| Tensile strain (maximum tensile Strain) [%GL] | 14.6 | 16.3 | 48.5 |

**(4) Viscosity measurement of atelocollagen**

It was investigated whether the viscosity of atelocollagen was changed according to the ratio of $\beta$ chain and $\alpha2$ chain of atelocollagen.

Specifically, the atelocollagen solutions of Examples 1, 42, and 31 and the atelocollagen of Comparative Example 2 were prepared to have a collagen concentration of 1.0% and a pH of 6.8 to 7.4 (neutral). Viscosity η (Pa s) at 10 °C of the prepared atelocollagen solution was measured using a rotary rheometer (TA instrument Ltd., ARES-G2). It was confirmed that Example 1 had a higher viscosity η than Comparative Example 2 under the shear rate 1 (1/s) condition. The viscosity η of Examples 1, 42 and 31 under the same conditions was decreased slightly as the ratio of the β chain to the α2 chain content was increased (see Table 5).

[TABLE 5]

| Section | Example 1 | Example 42 | Example 31 | Comparative Example 2 |
|---|---|---|---|---|
| β chain: α2 chain | 1.22:1 | 2.18:1 | 3.02:1 | 1.00:1 |
| Viscosity [Pa·s] | 2.46 | 2.19 | 1.92 | 0.776 |

## 6. Production of atelocollagen drug delivery system and analysis of physical properties

### (1) Production of solid atelocollagen drug delivery system

The high-purity atelocollagen prepared in the examples and comparative examples and a drug are mixed, poured into a circular silicone mold and lyophilized, thereby preparing a solid atelocollagen drug delivery system in which the drug is loaded (gemcitabine or [GRO-aptamer and gemcitabine conjugate]). For [GRO-aptamer and gemcitabine conjugate], a drug in which $(Gem)_2$ is bound to the 5' terminal of the $[TGG]_4$-TTG-$[TGG]_5$ sequence (SEQ ID NO: 1) was used (Gem: gemcitabine, $(Gem)_2$: 2 gemcitabines).

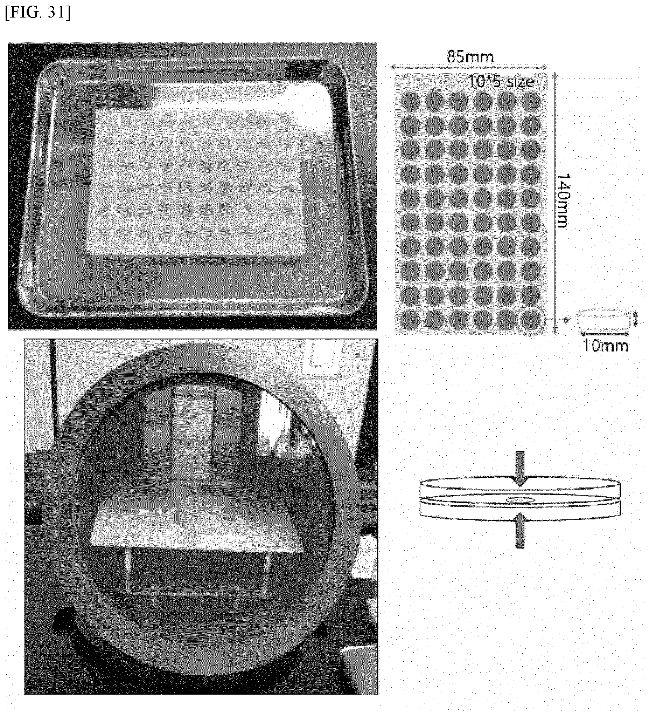

Specifically, after adding a drug (e.g., gemcitabine or [GRO-aptamer and gemcitabine conjugate]) to high-purity atelocollagen, this was uniformly mixed at room temperature for 30 minutes using a multi-mixer (SLRM-3, MYLAB). Thereafter, atelocollagen-drug mixture was dispensed in a constant amount in the circular silicone mold (upper photo in FIG. 31). The circular silicone mold, in which the atelocollagen-drug mixture was dispensed, was first frozen at -20 °C for 4 hours or more. After the primary freezing was completed, the sample was separated from the round silicone mold and transferred to a sterile dish or plate, and then frozen for 2 hours or more at -80 °C. After pre-freezing until the cold trap temperature of the lyophilizer reaches -80 °C, the atelocollagen sample was put into the lyophilizer and lyophilized for 16 hours or more (lower left photo in FIG. 31). The lyophilized solid atelocollagen drug delivery system was refrigerated at 4 °C.

### (2) Observation of structure of atelocollagen drug delivery system using scanning electron microscope (SEM)

Using a scanning electron microscope (SEM), it was confirmed whether the structure of the drug-loaded atelocollagen drug delivery system was changed according to the ratio of the β chain and α2 chain of atelocollagen. The atelocollagen solution in each of Example 1, Example 42, Example 31, Comparative Example 1 and Comparative Example 2 of the atelocollagen solution was mixed with [GRO-aptamer and gemcitabine conjugate] and the solution was lyophilized to prepare a circular sample having a diameter of 10 mm and a height of 5 mm. After coating the surface of the prepared sample using a platinum coating device, the coated sample was put into an SEM chamber, followed by applying a high voltage of 15.0 kV and measuring the surface, cross side, and bottom of the sample at magnifications of 50X, 100X, 200X, 500X, 1000X and 2000X. Thereafter, pore diameters of the prepared samples at the same magnification were measured, and the average diameter and standard deviation of the pores were calculated.

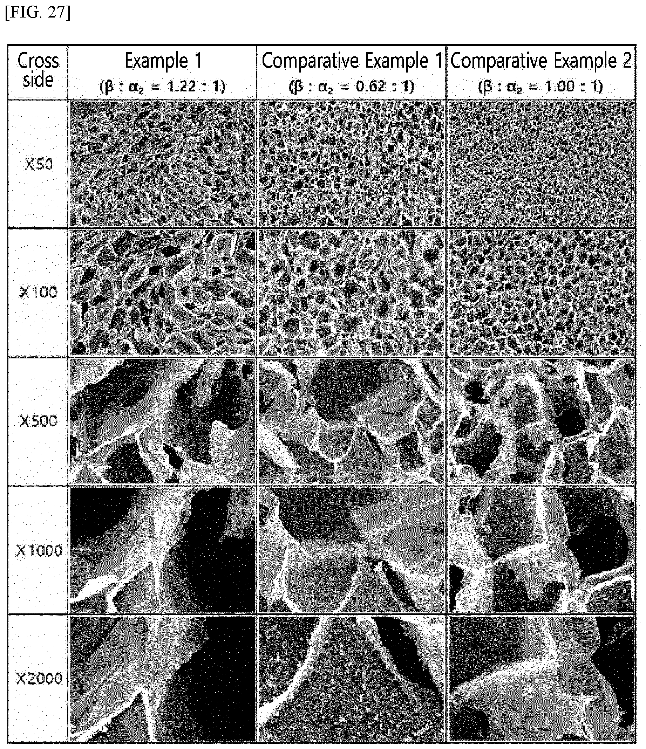

As a result of the measurement, it was confirmed that the atelocollagen sample of Example 1 loaded with the drug has pores opened on both sides, and the average diameter of the pores was decreased as the content of β chain relative to α2 was increased (see FIG. 27 and Table 6). On the other hand, Comparative Examples 1 and 2 contained pores with one wide side and the other narrow side. These results suggest that physical properties may vary depending on the content ratio of β chain and α2 chain.

[TABLE 6]

| Section | Example 1 | Comparative Example 1 | Comparative Example 2 |
|---|---|---|---|
| β chain: α2 chain | 1.22:1 | 0.62:1 | 1.00:1 |
| Average diameter of pore | 92.67 μm | Wide side: 132.69 μm<br>Narrow side: 39.38 μm | Wide side: 83.64 μm<br>Narrow side: 33.03μm |
| Standard deviation of pore diameter | 23.26 μm | Wide side: 28.04 μm<br>Narrow side: 29.25 μm | Wide side: 19.15 μm<br>Narrow side: 7.93 μm |

(continued)

| Section | Example 1 | Comparative Example 1 | Comparative Example 2 |
|---|---|---|---|
| Pore type | Pore open on both sides | Pore with one wide side and the other narrow side | Pore with one wide side and the other narrow side |

**(3) Drug release test of atelocollagen drug delivery system**

It was investigated whether the drug release pattern of the atelocollagen drug delivery system was changed according to the ratio of the $\beta$ chain and $\alpha2$ chain of atelocollagen.

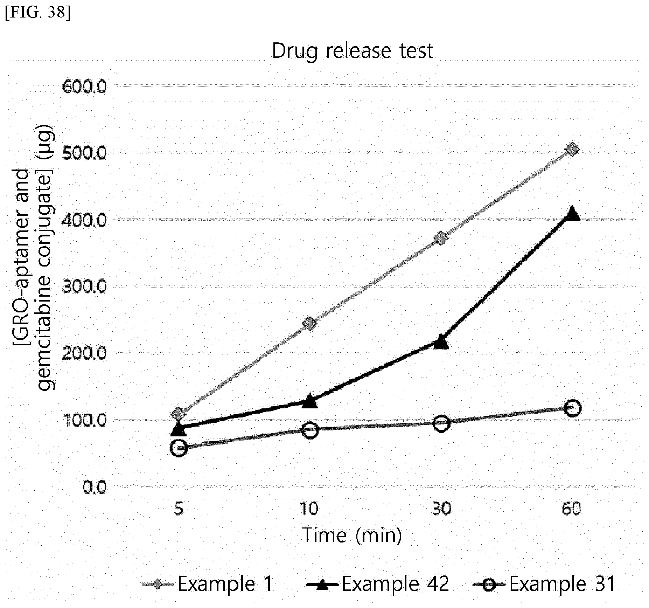

By the same method as the above "6. (1) Production of solid atelocollagen drug delivery system" but, after lyophilization, the atelocollagen sample was compressed in the form of a patch using an acrylic plate (lower right photo in FIG. 31). The patch type atelocollagen drug delivery system was prepared to load 2 mg of the [GRO-aptamer and gemcitabine conjugate] per one patch type atelocollagen drug delivery system. More specifically, each of the atelocollagen solutions in Examples 1, 42 and 31 was mixed with [GRO-aptamer and gemcitabine conjugate] to prepare a solution, followed by lyophilization to produce a patch type atelocollagen drug delivery system having a diameter of 10 mm. Following this, the patch type atelocollagen drug delivery system was added to an eluate, and then, the drug eluted at 37 °C for each time period was measured. As a result of measuring the concentration of [GRO-aptamer and gemcitabine conjugate] released at Abs. 260 mm by UV, it was confirmed that the drug was gradually released as the $\beta$ chain content was increased (see FIG. 38).

**7. Tumor suppression and metastasis inhibitory effects of patch type atelocollagen drug delivery system**

Effects of inhibiting cancer cell death and metastasis by drug release were compared between the patch type drug delivery system loaded with the drug on the atelocollagen of Example 1 and the patch type drug delivery system loaded with the drug on the atelocollagen of Comparative Example 2.

As a result of the experiment, the patch type drug delivery system prepared from atelocollagen of Example 1 showed excellent effects of inhibiting pancreatic cancer and metastasis, whereas the patch type drug delivery system prepared from the atelocollagen of Comparative Example 2 had low pancreatic cancer inhibitory effects and metastasis to other tissues in the abdominal cavity, that is, metastasis occurred to the liver, kidney and peritoneum was confirmed. This is considered because MMP-2 and MMP-9 causing cancer metastasis may recognize well the atelocollagen of the present invention (examples) so that the patch type drug delivery system prepared with atelocollagen (examples) of the present invention may be degraded thus to easily release the drug, On the other hand, since MMP-2 and MMP-9 do not recognize the atelocollagen of the comparative examples well, the patch type drug delivery system prepared from the atelocollagen of the comparative examples is not decomposed well and thus the drug may not be easily released, therefore, cancer cell death and metastasis inhibitory effects are expected to be poor (see FIGS. 6 and 7).

Specific experimental methods and results that confirmed effects of releasing the loaded drug from the patch type atelocollagen drug delivery system, as well as tumor suppression and cancer metastasis effects of the patch type atelocollagen drug delivery system, will be described in the following (1) to (3).

**(1) Production of patch type atelocollagen drug delivery system**

The atelocollagen solution with neutral pH ("neutral pH atelocollagen solution") prepared in the above "2. (2) Preparation of atelocollagen solution with neutral pH" was mixed with the drug, poured into a circular silicone mold and lyophilized to obtain an atelocollagen patch loaded with a drug (gemcitabine or a drug bound with $(Gem)_2$ at the 5' terminal of SEQ ID NO: 1 [GRO-aptamer and gemcitabine conjugate]).

Specifically, after adding a drug (e.g., gemcitabine or [GRO-aptamer and gemcitabine conjugate]) to the neutral pH atelocollagen solution, this was uniformly mixed at room temperature for 30 minutes using a multi-mixer (SLRM-3, MYLAB). Thereafter, the atelocollagen-drug mixture was dispensed in a constant amount in a circular silicone mold (upper photo in FIG. 31). The circular silicone mold in which the atelocollagen-drug mixture was dispensed was first frozen at -20 °C for 4 hours or more. After the first freezing was completed, the sample was separated from the circular silicone mold and transferred to a sterile dish or plate, and then frozen for 2 hours or more at -80°C. After pre-freezing until the cold trap temperature of the lyophilizer reaches -80 °C, an atelocollagen sample was put into a lyophilizer and lyophilized for 16 hours or more (lower left photo in FIG. 31). After the lyophilized atelocollagen sample was compressed in the form of a patch using an acrylic plate (lower right photo in FIG. 31), it was put and sealed in an aluminum pouch. The packaged patch type atelocollagen drug delivery system was refrigerated at 4°C.

**(2) Drug release effect of patch type atelocollagen drug delivery system in pancreatic cancer cell lines**

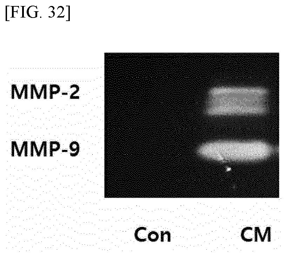

Pancreatic cancer cell line, BXPC-3, was cultured for 3 days to prepare a medium in which MMP-2 and MMP-9 were overexpressed (CM medium), as well as a medium in which MMP-2 and MMP-9 were not expressed (control medium). MMP-2 and MMP-9 activities were investigated using Zymography (see FIG. 32).

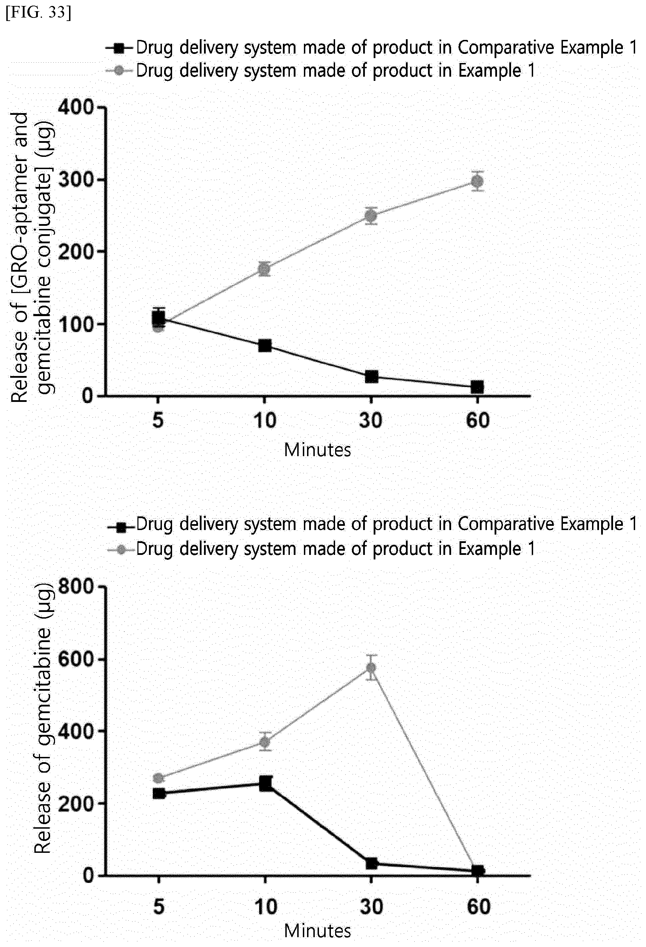

In the CM medium overexpressing MMP-2 and MMP-9 activities, it was investigated whether the drug ([GRO-aptamer and gemcitabine conjugate]), which is loaded from the atelocollagen drug delivery system of Example 1 and the atelocollagen drug delivery system of Comparative Example 2, is released well. A concentration of each of the released [GRO-aptamer and gemcitabine conjugate] and gemcitabine was measured with UV at Abs. 260 nm. As a result of the experiment, the patch type drug delivery system prepared using the atelocollagen of Example 1 was more soluble in the CM medium than in the control medium. Consequently, it was confirmed that [GRO-aptamer and gemcitabine conjugate] and gemcitabine contained in the drug delivery system are more actively released. Through this, it could be found that the atelocollagen of Example 1 was well recognized by MMP-2 and MMP-9. Further, it was confirmed that the atelocollagen drug delivery system of Example 1 may effectively release [GRO-aptamer and gemcitabine conjugate] and gemcitabine contained in the patch as compared to the atelocollagen drug delivery system of Comparative Example 2 in the CM medium (see FIG. 33).

**(3) Tumor suppression and metastasis inhibitory effects of patch type atelocollagen drug system after removal of residual cancer in a mouse model with pancreatic cancer**

**1) Tumor suppression and metastasis inhibitory effects of drug delivery system loaded with gemcitabine**

A patch type drug delivery system in which 0.12 mg of gemcitabine is loaded on atelocollagen of Example 1 (hereinafter, Gem/AC [Example 1] patch type drug delivery system) and a patch type drug delivery system in which 0.12 mg of gemcitabine is loaded on atelocollagen of Comparative Example 2 (hereinafter, Gem/AC [Comparative Example 2] patch type drug delivery system) were transplanted into orthotopic pancreatic cancer mice, respectively, so as to observe whether the residual tumor was suppressed and metastasis to other organs in the abdominal cavity was inhibited.

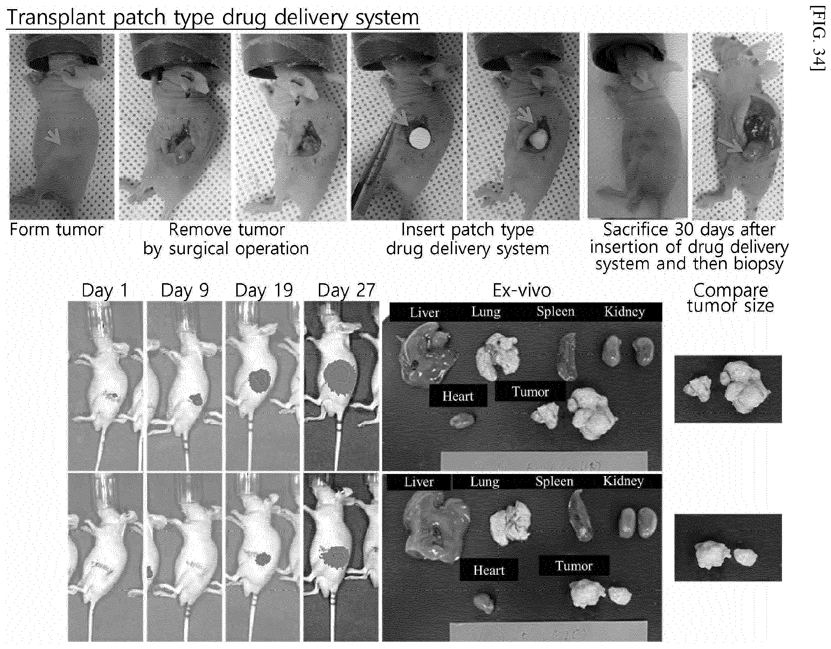

Specifically, Balb/c-nude mice (male, 6 weeks old, 21 mice) and 25 $\mu$l of $5 \times 10^5$ B $\times$ PC3 cancer cell line (BXPC-3-Luc cells) expressing luciferase by substitution of a specific vector were prepared. Each pancreatic cancer mouse model was prepared by dissecting the abdominal region of the anesthetized mouse, removing the pancreas, and then injecting BXPC-3-Luc cells into the pancreas. After 2 weeks, luciferase imaging was conducted to confirm the tumor. Tumors were measured using luciferin. In order to reduce autofluorescence, non-fluorescent feed was provided to the pancreatic cancer mouse models one week before imaging. After removing pancreatic cancer by incision in the abdominal region of the anesthetized mouse, Gem/AC [Comparative Example 2] patch type drug delivery system or Gem/AC [Example 1] patch type drug delivery system was implanted in the remaining tumor site. Tumor size and whether metastasis occurs were confirmed using luciferase imaging. After 30 days have elapsed since the insertion of each drug delivery system, the experimental animals were checked *ex-vivo* for tumor and metastasis to organs. As a result, compared to the Gem/AC [Comparative Example 2] patch type drug delivery system, the Gem/AC [Example 1] patch type drug delivery system exhibited superior tumor suppression and metastasis inhibitory effects (see FIG. 34).

**2) Tumor suppression and metastasis inhibitory effects of [GRO-Aptamer and gemcitabine conjugate] loaded drug delivery system**

By the same method as the above "7. (3)-1) tumor suppression and metastasis inhibitory effect of drug delivery system loaded with gemcitabine" but using [GRO-aptamer and gemcitabine conjugate] as the loaded drug, suppression of residual tumors and whether metastasis to other organs in the abdominal cavity is inhibited or not were observed.

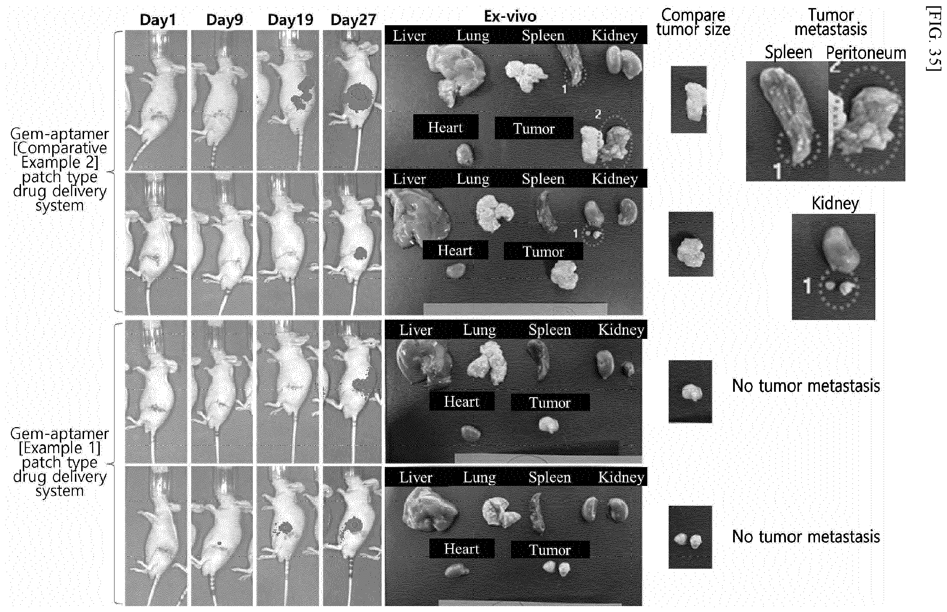

More specifically, the patch type drug delivery system in which 2 mg of [GRO-aptamer and gemcitabine conjugate] was loaded on atelocollagen of Example 1 (hereinafter, Gem-aptamer [Example 1] patch type drug delivery system) and the patch type drug delivery system in which 2 mg of [GRO-aptamer and gemcitabine conjugate] was loaded on atelocollagen of Comparative Example 2 (hereinafter, Gem-aptamer [Comparative Example 2] patch type drug delivery system) were transplanted into orthotopic pancreatic cancer mice, respectively, followed by observing suppression of residual tumors and whether metastasis to other organs in the abdominal cavity is inhibited or not. As a result, in the mice transplanted with the Gem-aptamer [Comparative Example 2] patch type drug delivery system, cancer metastasis to the spleen, peritoneum, and kidneys, etc. was exhibited. On the other hand, in the mice transplanted with the Gem-aptamer [Example 1] patch type drug delivery system, it was confirmed that there was no intraperitoneal metastasis and the tumor size was also small (see FIG. 35).

**8. Tumor suppression and metastasis inhibitory effects of sol-gel-type atelocollagen drug delivery system (drug-loaded atelocollagen sol-gel)**

**(1) Production of sol-gel type atelocollagen drug delivery system**

After adding the drug (gemcitabine or [GRO-aptamer and gemcitabine conjugate]) to 100 μL of the neutral pH atelocollagen solution at a concentration of 0.5% to 3.0%, this was uniformly mixed at room temperature or 30 minutes using a multi-mixer (SLRM-3, MYLAB). Alternatively, 100 μL of the neutral pH atelocollagen solution at a concentration of 0.5% to 3.0% and the drug were added to two syringes, respectively, and then the two syringes were connected and evenly mixed through piston reciprocation.

**(2) Tumor suppression and metastasis inhibitory effects of sol-gel-type atelocollagen drug delivery system after removal of residual cancer in a mouse model with pancreatic cancer**

**1) Tumor suppression and metastasis inhibitory effects of drug delivery system loaded with gemcitabine**

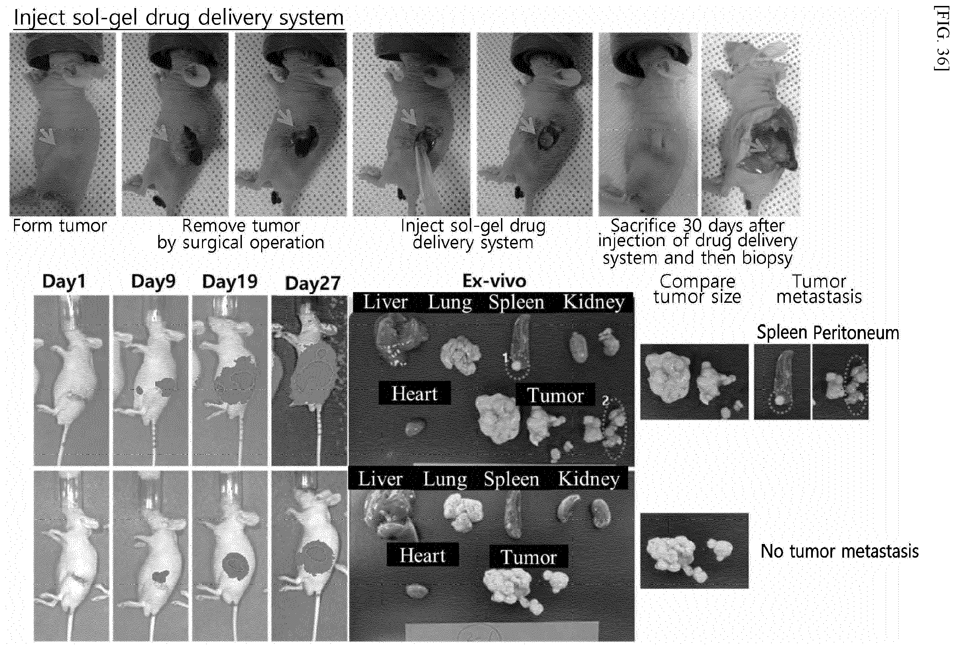

By the same method as the above "7.(3)-1) Tumor suppression and metastasis inhibitory effects of drug delivery system loaded with gemcitabine," but injecting a drug delivery system in which 0.12 mg of gemcitabine was loaded in the sol-gel made of the atelocollagen of Example 1 (hereinafter, Gem/AC [Example 1] sol-gel drug delivery system) and a drug delivery system in which 0.12 mg of gemcitabine was loaded in the sol-gel made of the atelocollagen of Comparative Example 2 (hereinafter, Gem/AC [Comparative Example 2] sol-gel drug delivery system) into orthotopic pancreatic cancer mice, respectively, followed by observing suppression of residual tumors and whether metastasis to other organs in the abdominal cavity is inhibited or not. As a result, in the mice injected with the Gem/AC [Comparative Example 2] sol-gel drug delivery system, cancer metastasis to the spleen, peritoneum, etc. was exhibited. On the other hand, in the mice injected with the Gem/AC [Example 1] sol-gel drug delivery system, it was confirmed that there was no intraperitoneal metastasis at all (see FIG. 36).

**2) Tumor suppression and metastasis effects of [GRO-aptamer and gemcitabine conjugate] loaded drug delivery system**

By the same method as the above "8. (2)-1) Tumor suppression and metastasis inhibitory effects of drug delivery system loaded with gemcitabine," but using [GRO-aptamer and gemcitabine conjugate] as the loaded drug, suppression of residual tumors and whether metastasis to other organs in the abdominal cavity occurs were observed.

A drug delivery system to which 2 mg of [GRO-aptamer and gemcitabine conjugate] was loaded in the sol-gel made of the atelocollagen (hereinafter, Gem/aptamer [Example 1] sol-gel drug delivery system) and a drug delivery system in which 2 mg of [GRO-aptamer and gemcitabine conjugate] was loaded in the sol-gel made of the atelocollagen of Comparative Example 2 (hereinafter, Gem/aptamer [Comparative Example 2] sol-gel drug delivery system) were injected into orthotopic pancreatic cancer mice, respectively, followed by observing suppression of residual tumors and whether metastasis to other organs in the abdominal cavity is inhibited or not.

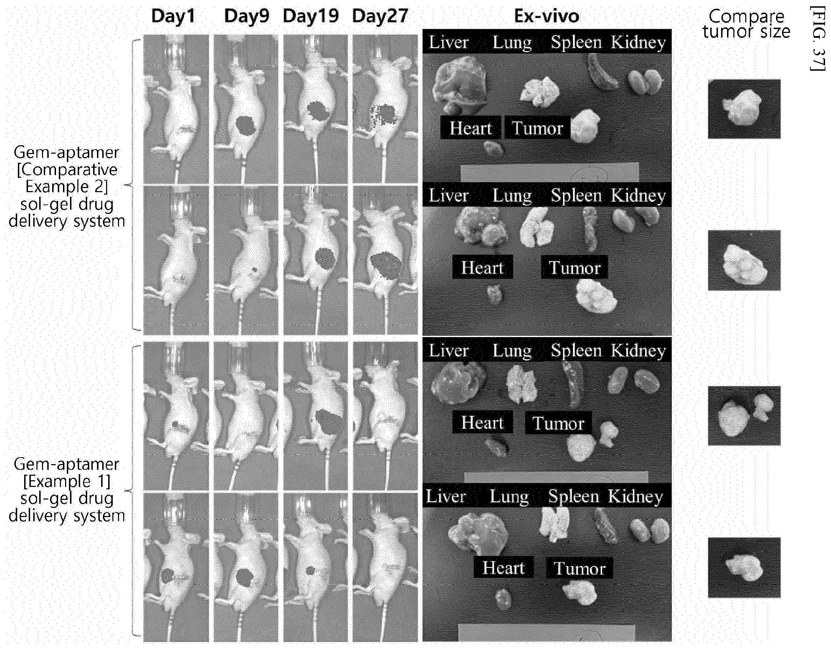

As a result, it was confirmed that the tumor size is smaller in the mice injected with Gem/aptamer [Example 1] sol-gel drug delivery system compared to the mice to which the Gem/aptamer [Comparative Example 2] sol-gel drug delivery system was injected (see FIG. 37).

[Description of Referential Numerals]

1,2: Area of ridge with HPLC peak
10, 20, 30, 40: HPLC peak
a1, a2, a3: Inflection point between ridge and ridge with HPLC peaks
b: Baseline of HPLC chromatogram
t10, t20: Elution time corresponding to the HPLC peak
ta1, ta2, ta3: Elution time corresponding to the inflection point between the ridge with two HPLC peaks
tb1, tb2: Elution time corresponding to the intersection of the baseline and the ridge having the HPLC peak

**Claims**

1. An atelocollagen, comprising:

   an $\alpha$ chain containing a repeat unit of Structural Formula 1 or Structural Formula 2 below; a $\beta$ chain as a dimer of the $\alpha$ chain; and a $\gamma$ chain as a trimer of the $\alpha$ chain,
   wherein, when analyzed by high-performance liquid chromatography (HPLC), a peak area ($S_\beta$) for the $\beta$ chain is larger than a second peak area for the $\alpha$ chain ($S_{\alpha2}$):

   [Formula 1]          Gly-Pro-X

   (in Structural Formula 1, Gly is glycine, Pro is proline, and X is an amino acid residue other than glycine and proline)

   [Structural Formula 2]          Gly-Y-Hyp

   (in Structural Formula 2, Gly is glycine, Y is an amino acid residue other than glycine and hydroxyproline, and Hyp is hydroxyproline).

2. The atelocollagen according to claim 1, wherein the $\alpha$ chain is at least one among an $\alpha$1 chain or an $\alpha$2 chain, the $\beta$ chain is a dimer of the $\alpha$1 chain and $\alpha$1 chain, a dimer of the $\alpha$1 chain and $\alpha$2 chain, or a dimer of the $\alpha$2 chain and $\alpha$2 chain.

3. The atelocollagen according to claim 1, wherein the $\alpha$ chain is at least one of $\alpha$1 chain or $\alpha$2 chain, and the $\gamma$ chain is a trimer of the $\alpha$1 chain, $\alpha$1 chain and $\alpha$2 chain.

4. The atelocollagen according to claim 1, wherein the $S_\beta$ and $S_{\alpha2}$ satisfy the following Equation 1:

$$[\text{Equation 1}]$$

$$1.1 \leq S_\beta / S_{\alpha2} \leq 5$$

5. The atelocollagen according to claim 1, wherein the $S_\beta$ satisfies the following Equation 2:

$$[\text{Equation 2}]$$

$$0.2 \leq S_\beta / (S_{\alpha1} + S_{\alpha2} + S_\beta + S_\gamma) \leq 0.5$$

(wherein $S_{\alpha1}$ is a first peak area for the $\alpha$ chain and $S_\gamma$ is a peak area for the $\gamma$ chain).

6. The atelocollagen according to claim 1, wherein the $\alpha$ chain has a molecular weight of 100 kDa to 150 kDa.

7. The atelocollagen according to claim 1, wherein the high-performance liquid chromatography (HPLC) analysis is performed under conditions shown in Table 1 below:

[TABLE 1]

| Column | Reverse phase C18 column filled with silica fixed phase having octadecyl functional group bonded thereto |
|---|---|
| Mobile phase A buffer | 0.1% trifluoroacetic acid (TFA) in distilled water |
| Mobile phase B buffer | 0.1% trifluoroacetic acid in acetonitrile |
| Detection wavelength | UV 220 nm |
| Flow rate | 1 ml/min |

8. The atelocollagen according to claim 7, wherein the $S_\beta$ is a value obtained by integrating peaks at an elution time of 13.0 to 13.7 minutes in a high-performance liquid chromatography (HPLC) graph.

9. The atelocollagen according to claim 7, wherein the $S_{\alpha2}$ is a value obtained by integrating peaks at an elution time of 13.7 to 14.2 minutes in a high-performance liquid chromatography (HPLC) chromatogram.

10. The atelocollagen according to claim 1, wherein the atelocollagen is obtained by treating a mammalian-derived dermis with a protease at 1 to 10 °C for 0.5 to 12 hours.

11. The atelocollagen according to claim 10, wherein the protease is used for treatment in an amount of $0.1 \times 10^7$ unit to $3.0 \times 10^7$ unit per 100 g of dermis.

12. The atelocollagen according to claim 1, wherein the atelocollagen is prepared by a method including:

    treating a mammalian-derived dermis with a protease;
    adding a basic solution to the dermis treated with the protease and adjusting pH in a range of 8 to 9, so as to inactivate the protease; and
    pH adjustment step of treating the sample, in which the protease was inactivated, with an acidic solution to regulate the pH of the sample to 3 to 4.

13. A drug delivery system, comprising: the atelocollagen according to any one of claims 1 to 12; and a drug loaded in the atelocollagen.

14. The drug delivery system according to claim 13, wherein the atelocollagen is a structure having a plurality of pores, wherein the pore has an average diameter of 10 to 100 $\mu$m.

15. The drug delivery system according to claim 13, wherein the drug delivery system is transplanted in or injected into a remaining cancer site after surgery.

16. A cancer metastasis inhibitor, comprising: the atelocollagen according to any one of claims 1 to 12; and an anticancer agent loaded in the atelocollagen.

17. The cancer metastasis inhibitor according to claim 16, wherein the cancer is pancreatic cancer, breast cancer, liver cancer, colorectal cancer, ovarian cancer, head and neck cancer or glioblastoma.

18. The cancer metastasis inhibitor according to claim 16, wherein the cancer metastasis inhibitor is transplanted in or injected into the remaining cancer site after surgery.

19. The cancer metastasis inhibitor according to claim 16, wherein the anticancer agent is a low molecular weight compound, high molecular weight compound, nucleic acid, peptide, protein or aptamer.

[FIG. 1]

[FIG. 2]

[FIG. 3]

[FIG. 4]

```
┌─────────────────────────────────────────────────────────┐
│                    Fragment pig skin                     │
└─────────────────────────────────────────────────────────┘
                            │
                            ▼
┌─────────────────────────────────────────────────────────┐
│                      Obtain dermis                       │
└─────────────────────────────────────────────────────────┘
                            │
                            ▼
┌─────────────────────────────────────────────────────────┐
│          Homogenize dermis while adding acetic acid      │
└─────────────────────────────────────────────────────────┘
                            │
                            ▼
┌─────────────────────────────────────────────────────────┐
│          Treat homogenized dermis sample with pepsin     │
└─────────────────────────────────────────────────────────┘
                            │
                            ▼
┌─────────────────────────────────────────────────────────┐
│            Inactivate pepsin from dermis sample          │
└─────────────────────────────────────────────────────────┘
                            │
                            ▼
┌─────────────────────────────────────────────────────────┐
│       Adjust pH of sample containing inactivated pepsin  │
└─────────────────────────────────────────────────────────┘
                            │
                            ▼
┌─────────────────────────────────────────────────────────┐
│                   Collect supernatant                    │
│        from pH-adjusted dermis sample and salt out       │
└─────────────────────────────────────────────────────────┘
                            │
                            ▼
┌─────────────────────────────────────────────────────────┐
│       Wash precipitate obtained during salting out       │
└─────────────────────────────────────────────────────────┘
                            │
                            ▼
┌─────────────────────────────────────────────────────────┐
│   Dissolve washed precipitate in solvent and filter solution │
└─────────────────────────────────────────────────────────┘
```

[FIG. 5]

[FIG. 6]

[FIG. 7]

[FIG. 8]

[FIG. 9]

[FIG. 10]

[FIG. 11]

| | Example 1 | Comparative Example 1 | Comparative Example 2 |
|---|---|---|---|
| γ chain | 8.16 | 7.00 | 4.70 |
| α1 chain | 50.76 | 59.84 | 54.29 |
| β chain | 23.29 | 12.71 | 20.39 |
| α2 chain | 17.79 | 20.45 | 20.29 |

[FIG. 12]

[FIG. 13]

Difference in chain content (%) according to different implement steps

| | Comparative Example 3 | Example 2 | Example 3 | Example 4 | Example 5 | Example 1 |
|---|---|---|---|---|---|---|
| γ chain | 7.29 | 4.20 | 4.06 | 5.02 | 5.47 | 6.13 |
| α1 chain | 56.14 | 52.64 | 52.63 | 51.02 | 50.59 | 48.39 |
| β chain | 14.49 | 26.74 | 25.82 | 25.39 | 25.91 | 25.01 |
| α2 chain | 22.09 | 16.42 | 17.50 | 18.56 | 18.02 | 20.47 |

[FIG. 14]

Difference in chain content (%) according
to pepsin treatment temperature and time

Legend: ▨ γ chain   ▥ α1 chain   ■ β chain   ▤ α2 chain

| Pepsin treatment at 4 °C | Example 11 | Example 12 | Example 13 | Example 14 | Comparative Example 5 | Comparative Example 4 |
|---|---|---|---|---|---|---|
| γ chain | 3.72 | 4.00 | 5.53 | 5.14 | 5.45 | 4.34 |
| α1 chain | 53.25 | 54.46 | 55.07 | 53.23 | 56.28 | 56.58 |
| β chain | 25.18 | 22.76 | 20.46 | 22.24 | 16.16 | 18.62 |
| α2 chain | 17.85 | 18.78 | 18.94 | 19.39 | 22.12 | 20.46 |

[FIG. 15]

## Difference in chain content (%) according to pepsin treatment temperature and time

| Pepsin treatment at RT | Example 15 | Comparative Example 12 | Comparative Example 13 | Comparative Example 14 | Comparative Example 15 | Comparative Example 6 |
|---|---|---|---|---|---|---|
| γ chain | 3.39 | 5.35 | 6.31 | 5.83 | 5.31 | 8.42 |
| α1 chain | 53.23 | 55.35 | 57.16 | 59.93 | 60.86 | 57.14 |
| β chain | 25.66 | 18.24 | 14.91 | 11.95 | 10.95 | 11.90 |
| α2 chain | 17.72 | 21.07 | 21.63 | 22.30 | 22.87 | 22.55 |

[FIG. 16]

Difference in chain content (%) according to different implement steps

| Pepsin treatment at 4 °C | Comparative Example 4 | Example 7 | Example 8 | Example 9 | Example 10 | Example 6 |
|---|---|---|---|---|---|---|
| γ chain | 4.34 | 4.57 | 6.75 | 5.01 | 4.22 | 4.99 |
| α1 chain | 56.58 | 59.51 | 54.98 | 49.72 | 49.40 | 48.64 |
| β chain | 18.62 | 23.84 | 23.99 | 24.64 | 26.00 | 26.13 |
| α2 chain | 20.46 | 12.08 | 14.28 | 20.63 | 20.39 | 20.24 |

[FIG. 17]

Difference in chain content (%) according to pepsin treatment temperature and time

| Pepsin treatment at RT | Comparative Example 6 | Comparative Example 7 | Comparative Example 8 | Comparative Example 9 | Comparative Example 10 | Comparative Example 11 |
|---|---|---|---|---|---|---|
| γ chain | 8.42 | 10.56 | 10.48 | 6.96 | 5.03 | 9.40 |
| α1 chain | 57.14 | 60.15 | 61.23 | 54.71 | 55.99 | 52.23 |
| β chain | 11.90 | 10.56 | 11.03 | 13.29 | 14.00 | 14.04 |
| α2 chain | 22.55 | 18.73 | 17.26 | 25.03 | 24.98 | 24.34 |

[FIG. 18]

## Difference in chain content (%) according to different implement steps

☒ γ chain    ▥ α1 chain    ■ β chain    ▤ α2 chain

| Pepsin amount –predetermined amount treatment(1.5 hr) | Example 16 | Example 17 | Example 18 | Example 19 | Example 20 |
|---|---|---|---|---|---|
| γ chain | 9.13 | 7.28 | 4.62 | 4.07 | 8.24 |
| α1 chain | 38.66 | 41.74 | 41.54 | 39.77 | 37.57 |
| β chain | 42.91 | 43.63 | 43.07 | 44.98 | 42.68 |
| α2 chain | 9.30 | 7.35 | 10.77 | 11.18 | 11.51 |

[FIG. 19]

## Difference in chain content (%) according to different implement steps

☒ γ chain    ▥ α1 chain    ■ β chain    ▤ α2 chain

| Pepsin amount –half amount treatment(1.5hr) | Example 26 | Example 27 | Example 28 | Example 39 | Example 30 | Example 31 |
|---|---|---|---|---|---|---|
| γ chain | 5.11 | 8.91 | 4.12 | 4.83 | 4.71 | 5.41 |
| α1 chain | 44.24 | 36.43 | 42.11 | 38.74 | 40.48 | 39.40 |
| β chain | 38.07 | 43.24 | 44.43 | 44.40 | 42.72 | 41.46 |
| α2 chain | 12.58 | 11.42 | 9.34 | 12.02 | 12.09 | 13.73 |

[FIG. 20]

## Difference in chain content (%) according to salting-out time

| Pepsin amount – predetermined amount treatment (1.5 hr) | Example 17 | Example 21 | Example 22 | Example 23 | Example 24 | Example 25 |
|---|---|---|---|---|---|---|
| γ chain | 7.28 | 7.15 | 6.29 | 5.50 | 3.80 | 4.62 |
| α1 chain | 41.74 | 36.17 | 37.25 | 41.66 | 41.06 | 41.54 |
| β chain | 43.63 | 44.02 | 44.59 | 41.06 | 44.46 | 43.07 |
| α2 chain | 7.35 | 12.66 | 11.88 | 11.79 | 10.67 | 10.77 |

[FIG. 21]

## Difference in chain content (%) according to salting-out time

Legend: γ chain, α1 chain, β chain, α2 chain

| Pepsin amount –half amount treatment(1.5 hr) | Example 28 | Example 32 | Example 33 | Example 34 | Example 35 | Example 36 |
|---|---|---|---|---|---|---|
| γ chain | 4.12 | 6.52 | 4.94 | 4.17 | 4.54 | 4.83 |
| α1 chain | 42.11 | 35.16 | 38.18 | 41.12 | 39.43 | 38.74 |
| β chain | 44.43 | 45.72 | 45.52 | 44.69 | 45.25 | 44.40 |
| α2 chain | 9.34 | 12.61 | 11.36 | 10.03 | 10.79 | 12.02 |

[FIG. 22]

## Difference in chain content (%) according to pepsin treatment time

|  | Example 11 | Example 43 | Example 44 |
|---|---|---|---|
| γ chain | 3.72 | 5.05 | 6.65 |
| α1 chain | 53.25 | 47.60 | 49.87 |
| β chain | 25.18 | 37.36 | 30.42 |
| α2 chain | 17.85 | 10.00 | 13.06 |

[FIG. 23]

## Difference in chain content (%) according to salting-out time

| salting out (pellet) | Example45 | Example46 | Example47 | Example48 | Example49 | Example50 | Example51 |
|---|---|---|---|---|---|---|---|
| γ chain | 7.81 | 9.93 | 5.15 | 5.39 | 5.94 | 5.77 | 7.34 |
| α1 chain | 47.02 | 44.27 | 49.60 | 49.83 | 45.84 | 47.85 | 47.14 |
| β chain | 33.75 | 28.79 | 32.25 | 31.54 | 35.26 | 34.61 | 31.30 |
| α2 chain | 11.42 | 17.01 | 13.00 | 13.24 | 12.96 | 11.77 | 14.22 |

[FIG. 24]

## Difference in chain content (%) according to different implement steps

| | Example 37 | Example 38 | Example 39 | Example 40 | Example 41 | Example 42 |
|---|---|---|---|---|---|---|
| γ chain | 6.65 | 5.15 | 5.78 | 7.34 | 3.08 | 3.32 |
| α1 chain | 49.87 | 52.77 | 51.46 | 47.14 | 49.05 | 48.86 |
| β chain | 30.42 | 33.55 | 32.53 | 31.30 | 32.68 | 32.77 |
| α2 chain | 13.06 | 8.53 | 10.23 | 14.22 | 15.19 | 15.04 |

[FIG. 25]

[FIG. 26]

[FIG. 27]

| Cross side | Example 1<br>($\beta : \alpha_2 = 1.22 : 1$) | Comparative Example 1<br>($\beta : \alpha_2 = 0.62 : 1$) | Comparative Example 2<br>($\beta : \alpha_2 = 1.00 : 1$) |
|---|---|---|---|
| X50 | | | |
| X100 | | | |
| X500 | | | |
| X1000 | | | |
| X2000 | | | |

[FIG. 28]

## Graph is an arbitrary string

Tension testResult

| Testing machine name | MCT | Type of test | Tension | |
|---|---|---|---|---|
| Load cell load rating | 500 N | Test speed | 10.0 mm/min | |
| PointData(Load) | 0 N | PointData(Elong) | 0 mm | |
| Distance between jaws | 8.94 mm | Breaking point measu | 0.5 N | |
| Origin of elongation | Initial load point | Initial load value | 0.1 N | |
| Elong adjust | No | Slope Analysis | 1 To | 100 N |
| Pitch | 5 N | Invalid amplitude level | 1 N | |
| Test start point | Initial load point | Test end point | From initial load point | 1000 mm |
| Sample name | dTS IN-A1 | Operator | | |

| TestID=675<br>Test No | Maximum poin<br>Load<br>N | Maximum poin<br>Stress<br>MPa | Maximum_poin<br>Elongation<br>mm | Maximum_poin<br>Strain<br>%GL | Maximum_poin<br>Energy<br>N-m |
|---|---|---|---|---|---|
| 1 | 0.5137 | 0.1071 | 1.3042 | 14.588 | 0.0004 |
| Average | 0.5137 | 0.1071 | 1.3042 | 14.588 | 0.0004 |
| Max. | 0.5137 | 0.1071 | 1.3042 | 14.588 | 0.0004 |
| Min. | 0.5137 | 0.1071 | 1.3042 | 14.588 | 0.0004 |
| Range | 0.0000 | 0.0000 | 0.0000 | 0.0000 | 0.0000 |

[FIG. 29]

## Graph is an arbitrary string

Tension testResult

| Testing machine name | MCT | Type of test | Tension |
|---|---|---|---|
| Load cell load rating | 500 N | Test speed | 10.0 mm/min |
| PointData(Load) | 0 N | PointData(Elong) | 0 mm |
| Distance between jaws | 9.03 mm | Breaking point measu | 0.5 N |
| Origin of elongation | Initial load point | Initial load value | 0.1 N |
| Elong adjust | No | Slope Analysis | 1 To 100 N |
| Pitch | 5 N | Invalid amplitude leve | 1 N |
| Test start point | Initial load point | Test end point | From initial load point 1000 mm |

| Sample name | dTS IN-B1 | Operator | |
|---|---|---|---|

| TestID=678 | Maximum poin | Maximum poin | Maximum_poin | Maximum_poin | Maximum_poin |
|---|---|---|---|---|---|
| | Load | Stress | Elongation | Strain | Energy |
| Test No | N | MPa | mm | %GL | N-m |
| 1 | 1.3858 | 0.2745 | 4.0150 | 44.463 | 0.0030 |
| Average | 1.3858 | 0.2745 | 4.0150 | 44.463 | 0.0030 |
| Max. | 1.3858 | 0.2745 | 4.0150 | 44.463 | 0.0030 |
| Min. | 1.3858 | 0.2745 | 4.0150 | 44.463 | 0.0030 |
| Range | 0.0000 | 0.0000 | 0.0000 | 0.0000 | 0.0000 |

[FIG. 30]

## Graph is an arbitrary string

Tension testResult

| Testing machine name | MCT | Type of test | Tension |
|---|---|---|---|
| Load cell load rating | 500 N | Test speed | 10.0 mm/min |
| PointData(Load) | 0 N | PointData(Elong) | 0 mm |
| Distance between jaws | 9.24 mm | Breaking point measu | 0.5 N |
| Origin of elongation | Initial load point | Initial load value | 0.1 N |
| Elong adjust | No | Slope Analysis | 1 To          100 N |
| Pitch | 5 N | Invalid amplitude level | 1 N |
| Test start point | Initial load point | Test end point | From initial load point  1000 mm |

| Sample name | dTS_IN-C1 | Operator | |
|---|---|---|---|

| TestID=680 | Maximum poin | Maximum poin | Maximum_poin | Maximum_poin | Maximum_poin |
|---|---|---|---|---|---|
| | Load | Stress | Elongation | Strain | Energy |
| Test No | N | MPa | mm | %GL | N-m |
| 1 | 0.9582 | 0.2260 | 1.5058 | 16.297 | 0.0010 |
| Average | 0.9582 | 0.2260 | 1.5058 | 16.297 | 0.0010 |
| Max. | 0.9582 | 0.2260 | 1.5058 | 16.297 | 0.0010 |
| Min. | 0.9582 | 0.2260 | 1.5058 | 16.297 | 0.0010 |
| Range | 0.0000 | 0.0000 | 0.0000 | 0.0000 | 0.0000 |

[FIG. 31]

[FIG. 32]

[FIG. 33]

[FIG. 34]

Transplant patch type drug delivery system

Form tumor     Remove tumor     Insert patch type     Sacrifice 30 days after
by surgical operation     drug delivery system     insertion of drug delivery
system and then biopsy

Day 1    Day 9    Day 19    Day 27      Ex-vivo      Compare tumor size

[FIG. 35]

[FIG. 36]

EP 4 174 081 A1

## Inject sol-gel drug delivery system

Form tumor | Remove tumor by surgical operation | Inject sol-gel drug delivery system | Sacrifice 30 days after injection of drug delivery system and then biopsy

Day1 Day9 Day19 Day27 | Ex-vivo | Compare tumor size | Tumor metastasis

Liver Lung Spleen Kidney
Heart Tumor

Liver Lung Spleen Kidney
Heart Tumor

Spleen Peritoneum

No tumor metastasis

[FIG. 37]

EP 4 174 081 A1

[FIG. 38]

Drug release test

## INTERNATIONAL SEARCH REPORT

| | International application No. |
|---|---|
| | **PCT/KR2021/008154** |

**A. CLASSIFICATION OF SUBJECT MATTER**

**C07K 14/78**(2006.01)i; **A61K 47/42**(2006.01)i; **A61K 9/10**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

C07K 14/78(2006.01); A23J 3/34(2006.01); A61K 8/65(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아텔로콜라겐 (atelocollagen), 약물 전달 (drug delivery), 알파 사슬 (α-chain), 베타 사슬 (β-chain), 고성능 액체 크로마토그래피 (HPLC), 단백질 분해 효소 (protease), 펩신 (pepsin) 항암 (anti-cancer)

**C. DOCUMENTS CONSIDERED TO BE RELEVANT**

| Category* | Citation of document, with indication, where appropriate, of the relevant passages | Relevant to claim No. |
|---|---|---|
| A | GRØNLIEN, K. G. et al. Collagen from Turkey (Meleagris gallopavo) tendon: A promising sustainable biomaterial for pharmaceutical use. Sustainable Chemistry and Pharmacy. 2019, vol. 13, 100166, inner pp. 1-10 (published online:12 August 2019). See abstract, inner pages 1-8 and Figures 3 and 10. | 1-19 |
| A | LIN, K. Y. et al. Effects of pepsin digestion at different temperatures and times on properties of telopeptide-poor collagen from bird feet. Food Chemistry. 2006, vol. 94, pp. 621-625. See abstract, pages 621-624, Fig. 1 and Table 1. | 1-19 |
| A | DEYL, Z. et al. Advanced separation methods for collagen parent a-chains, their polymers and fragments. Journal of Chromatography B. 2000, vol. 739, pp. 3-31. See entire document. | 1-19 |
| A | ISHIHARA, Y. et al. A novel anti-cancer effect of atelocollagen-conjugated miR-520d-5p on pancreatic cancer cells in vitro and in a mouse xenograft model. Integrative Molecular Medicine. 2019, vol. 6, pp. 1-9 (publication date:26 April 2019). See entire document. | 1-19 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 October 2021** | **08 October 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2021/008154**

**C.     DOCUMENTS CONSIDERED TO BE RELEVANT**

| Category* | Citation of document, with indication, where appropriate, of the relevant passages | Relevant to claim No. |
|---|---|---|
| A | KR 10-1871395 B1 (HWANG, Jong Kyu) 26 June 2018 (2018-06-26)<br>See entire document. | 1-19 |

Form PCT/ISA/210 (second sheet) (July 2019)

## INTERNATIONAL SEARCH REPORT

| International application No. |
| --- |
| **PCT/KR2021/008154** |

**Box No. I**     **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13ter.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13ter.1(a)).

        ☐ on paper or in the form of an image file (Rule 13ter.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2021/008154**

| Patent document cited in search report | Publication date (day/month/year) | Patent family member(s) | Publication date (day/month/year) |
|---|---|---|---|
| KR 10-1871395 B1 | 26 June 2018 | None | |

Form PCT/ISA/210 (patent family annex) (July 2019)